(19) Europäisches Patentamt / European Patent Office / Office européen des brevets

(11) **EP 2 238 891 A1**

(12) **EUROPEAN PATENT APPLICATION**

(43) Date of publication:
**13.10.2010 Bulletin 2010/41**

(51) Int Cl.:
***A61B 5/00*** *(2006.01)*

(21) Application number: **10158110.6**

(22) Date of filing: **29.03.2010**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA ME RS**

(30) Priority: **30.03.2009 JP 2009083209**
**20.01.2010 JP 2010009941**

(71) Applicant: **SYSMEX CORPORATION**
**Kobe-shi,**
**Hyogo 651-0073 (JP)**

(72) Inventors:
- **Sato, Toshiyuki**
  **Kobe-shi Hyogo 651-0073 (JP)**
- **Okada, Seiki**
  **Kobe-shi Hyogo 651-0073 (JP)**

(74) Representative: **Paemen, Liesbet R.J. et al**
**De Clercq & Partners cvba**
**Edgard Gevaertdreef 10 a**
**9830 Sint-Martens-Latem (BE)**

(54) **Prediction method of concentration fluctuation of measurement target components in blood using area under blood concentration time curve, and device therefor**

(57) The invention provides A method for predicting a concentration fluctuation of a measurement target component in the blood, comprising steps of: obtaining an initial value of the amount relating to the measurement target component in a subject (S0); obtaining, as a first measurement value, a value of an area under the blood concentration time curve of the measurement target component during a first extraction period (S2,S3,S4,S9); obtaining, as a second measurement value, a value of an area under the blood concentration time curve of the measurement target component during a second extraction period (S6,S7,S8,S9); and predicting the concentration fluctuation of the measurement target component in the blood from the initial value, the first measurement value and the second measurement value (S10,S11), as well as a device for predicting a concentration fluctuation of a measurement target component in the blood.

*FIG. 13*

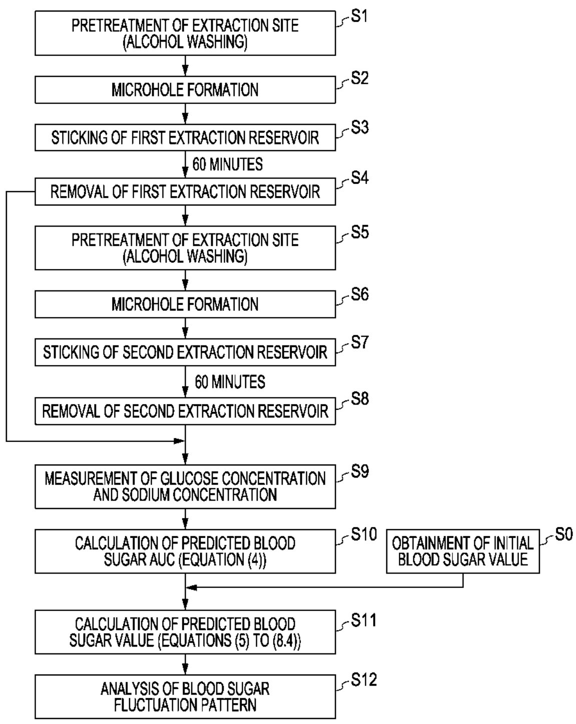


EP 2 238 891 A1

**Description**

FIELD OF THE INVENTION

The present invention relates to a prediction method of concentration fluctuation of measurement target components in the blood by obtaining an area under the blood concentration time curve and using the area under the blood concentration time curve, and also relates to a device therefor.

BACKGROUND ART

In the diagnosis of diabetic conditions, the maximum blood sugar value has been adopted as one criterion. It is therefore important to examine the blood sugar fluctuation so as to obtain the maximum blood sugar value and the time by which the maximum blood sugar value is reached from the beginning of a meal. For example, when insulin is administered to lower the blood sugar value, it is effective in therapies to ensure the peak of the blood sugar value and the peak of insulin acting on the living body at the same level. Therefore, insulin can be administered based on the time required for reaching the maximum blood sugar value if it is known to some extent. In addition, if the maximum blood sugar value of insulin is known, the dose of insulin can be adjusted according to it. As such, a detailed therapy depending on the characteristics of a patient (a subject) becomes possible by adjusting the dose of insulin and the timing of administration.

Fluctuations of blood sugar can be confirmed by drawing blood more than one time with predetermined time intervals and determining the blood sugar values. In addition, WO 9600110 discloses an iontophoretic sampling device equipped with an integrated sensor for monitoring non-intrusively a blood concentration of a target substance or a component. Using the device disclosed in WO 9600110, the fluctuations of blood sugar levels can also be examined. However, WO 9600110 does not describe a device for measuring the area under the blood concentration time curve. Moreover, the device disclosed in WO 9600110 does not mention the possibility of examining the fluctuations of blood sugar levels using the area under the blood concentration time curve.

SUMMARY OF THE INVENTION

The scope of the present invention is defined solely by the appended claims, and is not affected to any degree by the statements within this summary

An object of the present invention is to provide a prediction method of concentration fluctuation of a measurement target component in the blood, by obtaining an area under the blood concentration time curve and using the area under the blood concentration time curve, and also to provide a device therefor.

(1) A first aspect of the present invention is a method for predicting a concentration fluctuation of a measurement target component in the blood, comprising steps of:

obtaining an initial value of the amount of the measurement target component in a subject;
obtaining, as a first measurement value, the value of the area under the blood concentration time curve of the measurement target component, from the amount of the measurement target component contained in a tissue liquid extracted during a first extraction period from a microhole formed on the skin of the subject;
obtaining, as a second measurement value, the value of the area under the blood concentration time curve of the measurement target component, from the amount of the measurement target component contained in a tissue liquid extracted during a second extraction period from the microhole formed on the skin of the subject; and
predicting the concentration fluctuation of the measurement target component in the blood from the initial value, the first measurement value and the second measurement value.

(2) The method according to (1), wherein the second extraction period is a period after the completion of the first extraction period.
(3) The method according to (2), wherein the prediction step comprises:

obtaining a first blood concentration of the measurement target component at a first point from the first measurement value;
obtaining a second blood concentration of the measurement target component at a second point from the second measurement value; and
predicting the concentration fluctuation of the measurement target component in the blood from the initial value, the first blood concentration and the second blood concentration.

(4) The method according to (1), wherein the first extraction period and the second extraction period are started at the same time, and the second extraction period is longer than the first extraction period.
(5) The method according to (4), wherein the prediction step comprises:

obtaining a first blood concentration of the measurement target component at a first point from the first measurement value;
obtaining a second blood concentration of the measurement target component at a second point from the difference between the second measurement value and the first measurement value;
and
predicting the concentration fluctuation of the measurement target component in the blood from the initial value, the first blood concentration and the second blood concentration.

(6) The method according to any one of (1) to (5), wherein the measurement target component is glucose, and the concentration fluctuation of the measurement target component is a fluctuation of a blood glucose value.
(7) The method according to (6), wherein the fluctuation of a blood sugar value is expressed by each parameter of a prediction of the maximum blood sugar value, a prediction of the time reaching the maximum blood sugar value, and a blood sugar value fluctuation rate.
(8) The method according to any one of (1) to (7), wherein
the first measurement value is a value corrected by a concentration of sodium ion contained in the tissue liquid extracted during the first extraction period, and
the second measurement value is a value corrected by a concentration of sodium ion contained in the tissue liquid extracted during the second extraction period.
(9) The method according to any one of (1) to (8), wherein at least one of the first extraction period and the second extraction period is 60 minutes or more.
(10) The method according to any one of (1) to (9), wherein the sum of the first extraction period and the second extraction period is 120 minutes or more.
(11) The method according to any one of (1) to (9), wherein the second extraction period is 120 minutes or more.
(12) The method according to any one of (1) to (11), wherein the initial value is a blood concentration value of a measurement target component obtained by drawing blood from the subject.
(13) A second aspect of the present invention is a device for predicting a concentration fluctuation of a measurement target component in the blood, comprising:

an input part for inputting an initial value relating to the amount of a measurement target component of a subject;
a first collection member for retaining a tissue liquid extracted during a first extraction period from a microhole formed on the skin of the subject;
a second collection member for retaining a tissue liquid extracted during a second extraction period from the microhole formed on the skin of the subject;
a measurement part for measuring the amount of the measurement target component contained in the first collection member and the amount of the measurement target component contained in the second collection member; and
an analysis part configured for performing operations, comprising:

obtaining, as a first measurement value, the value of the area under the blood concentration time curve of the measurement target component during the first extraction period, from the amount of the measurement target component contained in the first collection member;
obtaining, as a second measurement value, the value of the area under the blood concentration time curve of the measurement target component during the second extraction period, from the amount of the measurement target component contained in the second collection member; and
predicting the concentration fluctuation of the measurement target component in the blood from the initial value, the first measurement value and the second measurement value.

(14) The device according to (13), wherein the second extraction period is a period after the completion of the first extraction period.
(15) The device according to (14), wherein the analysis part is configured for performing further operations, comprising:

obtaining a first blood concentration of a measurement target component at a first point from the first measurement value; and

obtaining a second blood concentration of a measurement target component at a second point from the second measurement value; wherein the concentration fluctuation of the measurement target component in the blood is predicted from the initial value, the first blood concentration and the second blood concentration.

(16) The device according to (13), wherein the first extraction period and the second extraction period are started at the same time, and the second extraction period is longer than the first extraction period.
(17) The device according to (16), wherein the analysis part is configured for performing further operations, comprising:

obtaining a first blood concentration of a measurement target component at a first point from the first measurement value; and
obtaining a second blood concentration of a measurement target component at a second point from a value obtained by subtracting the first measurement value from the second measurement value; wherein the concentration fluctuation of the measurement target component in the blood is predicted from the initial value, the first blood concentration and the second blood concentration.

(18) The device according to any one of (13) to (17), wherein the measurement target component is glucose and the concentration fluctuation of the measurement target component is a fluctuation of blood glucose value.

When using the above method (1) and the above device (13), an area under the blood concentration time curve can be obtained and a concentration fluctuation of a measurement target component in the blood can be predicted by using a value of the area under the blood concentration time curve.
When using the above method (6) and the above device (18), the blood sugar AUC can be obtained and the blood sugar fluctuation can be predicted. As a result, the fluctuation patterns of blood sugar (maximum blood sugar value, time reaching the maximum blood sugar value, blood sugar fluctuation rate and the like) can be predicted.
That is, according to the present invention, a prediction method of a concentration fluctuation of a measurement target component in the blood by obtaining the area under the blood concentration time curve and using the area under the blood concentration time curve, as well as a device therefor can be provided.

BRIEF DESCRIPTION OF THE DRAWINGS

Fig. 1 is an illustrative perspective view of one example of a puncture instrument used in the present invention;
Fig. 2 is a perspective view of a microneedle chip attached to the puncture instrument shown in Fig. 1;
Fig. 3 is an illustrative section view of the skin where microholes were formed with the puncture instrument;
Fig. 4 is an illustrative section view of one example of a collection member used in a prediction method of the present invention;
Fig. 5 is a graph showing the correlation between glucose permeability and a sodium ion extraction rate;
Fig. 6 is a graph showing the correlation between AUCBG and predicted AUCBG;
Fig. 7 is a graph showing the correlation between BG30min and predicted AUCBG(0-60);
Fig. 8 is a graph showing the correlation between BG90 min and predicted AUCBG(60-120);
Fig. 9 is a schematic view showing a prediction method of BG60min and BG120min;
Fig. 10 is a graph showing a fluctuation pattern A of blood sugar;
Fig. 11 is a graph showing a fluctuation pattern B of blood sugar;
Fig. 12 is a graph showing a fluctuation pattern C of blood sugar;
Fig. 13 is a flowchart of case 1;
Fig. 14 is an illustrative view when the collection member shown in Fig. 13 is used;
Fig. 15 is a view illustrating a collection method of an analyte in the gel using a collection tube;
Fig. 16 is a diagram showing a computer system configuration used for obtaining a fluctuation pattern of blood sugar;
Fig. 17 is a view illustrating a device A used for obtaining a fluctuation pattern of blood sugar;
Fig. 18 is a view illustrating a state that an extraction reservoir is set in a collection cartridge of a device B used for obtaining a fluctuation pattern of blood sugar;
Fig. 19 is a view illustrating a collection method of an analyte in the gel in the device B used for obtaining a fluctuation pattern of blood sugar;
Fig. 20 is a view illustrating a collection method of an analyte in the gel in the device B used for obtaining a fluctuation pattern of blood sugar;
Fig. 21 is an illustrative perspective view of a device C, a sensor chip and a collection member used for obtaining a fluctuation pattern of blood sugar;
Fig. 22 is an illustrative plane view of the device C shown in Fig. 21;
Fig. 23 is an illustrative side view of the device C shown in Fig. 21;
Fig. 24 is an illustrative plane view of the sensor chip shown in Fig. 21;
Fig. 25 is an illustrative side view of the sensor chip shown in Fig. 21;
Fig. 26 is a view illustrating the measurement procedure of the device C;
Fig. 27 is a view illustrating the measurement procedure of the device C; and
Fig. 28 is a flowchart of case 2.

DETAILED DESCRIPTION OF THE PREFERRED EMBODIMENTS

The preferred embodiments of the present invention will be described hereinafter with reference to the drawings.

A measurement target component as used herein refers to any component of interest to be measured in a body fluid, more particularly in the blood, including glucose, amino acids, enzyme substrates or products indicating a disease state or condition, other markers of disease states or conditions, drugs of abuse, therapeutic drugs, and electrolytes.

While the present invention is illustrated by the measurement of a blood component, it will be clear to the skilled person that the invention equally applies to measurements in other bodily fluids such as, but not limited to urine, spinal fluid, sweat etc. Accordingly, it will be understood that the reference to "blood concentration" can be replaced by body fluid concentration in particular embodiments.

The area under the blood concentration time curve (AUC) is known as a value reflecting the total amount of components which have circulated throughout the living body in a predetermined period of time. Concretely, the AUC means the area surrounded by a curve (blood concentration of *in vivo* components-time curve) drawn in the graph showing the blood concentration of a predetermined biological component with the passage of time and by a horizontal axis (time axis).

The blood sugar AUC means an area (unit: mg·h/dl) surrounded by a curve drawn in the graph showing the blood sugar value with the passage of time and by a horizontal axis. The blood sugar AUC can become an index used in determining the effect of insulin and oral drugs. For example, by measuring a value that reflects the total amount of glucose (blood sugar) which has circulated throughout the blood in a predetermined period of time after glucose loading (after a meal) using the blood sugar AUC, the total amount of glucose which has circulated throughout the body of the subject after glucose loading can be predicted. The total amount of glucose which has circulated throughout the body of the subject after glucose loading is extremely useful information to know how long a high blood glucose state due to the sugar loading has continued. For example, such a total amount of glucose becomes a clue to know the rate of insulin secretion after glucose loading. Moreover, it also becomes a clue to know the effect of insulin when an oral diabetes agent or insulin is administered.

However, the value of the blood sugar AUC has not been used enough in diabetes treatment up to now. Therefore, physicians as well as patients have difficulty understanding the diabetes only from the value of the blood sugar AUC. Accordingly, if the blood sugar AUC and the blood sugar fluctuation can be obtained and presented at the same time, it becomes easy to correlate the blood sugar AUC and the blood sugar fluctuation. In other words, physicians can correlate the blood sugar AUC with the blood sugar fluctuation at the time of OGTT which has been used conventionally. In addition, patients also can correlate the blood sugar AUC with the blood sugar value obtained by self-monitoring of the blood sugar. As a result, physicians as well as patients will more easily understand the blood sugar AUC and the result of the blood sugar fluctuation.

Hereinafter, embodiments of the prediction method and the device of the present invention will be explained in detail with reference to the attached drawings.

In the prediction method according to a particular embodiment of the invention, a means to form a microhole in the skin of the subject, an extraction reservoir to accumulate the tissue fluid extracted from the skin of the subject concerned, and a device capable of measuring a glucose concentration and a sodium ion concentration for correction are used to obtain a blood sugar value fluctuation pattern in the subject. First of all, one example of a puncture instrument that is a means for microhole formation and one example of an extraction reservoir used in the present invention will be explained respectively.

[PUNCTURE INSTRUMENT]

As shown in Figures 1 to 3, a puncture instrument 400 where a sterilized microneedle chip 500 is attached is a device for forming an extraction hole (microhole 601) for a tissue fluid on a skin 600 of a subject by contacting a microneedle 501 of the microneedle chip 500 with an epidermis (the skin 600 of the subject) of the living body. When the microhole 601 is formed with use of the puncture instrument 400, the microneedle 501 of the microneedle chip 500 has a size such that the microhole 601 stays in the epidermis and does not arrive at a dermis. As shown in Figure. 1, the puncture instrument 400 comprises an enclosure 401, a release button 402 installed on the surface of the enclosure 401, an array chuck 403 installed inside the enclosure 401 and a spring member 404. An opening (not shown) is formed in a lower part 401a of the enclosure 401. The spring member 404 has a function to push the array chuck 403 in the puncture direction. The array chuck 403 can attach the microneedle chip 500 to the bottom end. A plurality of the microneedles 501 are formed in the bottom surface of the microneedle chip 500. In addition, the puncture instrument 400 is configured to have a fixation mechanism (not shown) to fix the array chuck 403 pushed upward (anti-puncture direction) against the force of the spring member 404 so that the fixation of the array chuck 403 with the fixation mechanism is released by pushing down the release button 402 by a user (a subject) and the array chuck 403 moves in the puncture direction by the force of the spring member 404.

[EXTRACTION RESERVOIR]

As shown in Fig. 4, an extraction reservoir 300 has a structure that a gel 301 having a water retention property (not substantially including a sodium ion in a component) and being able to retain the tissue fluid extracted from the skin of the subject is supported by a supporting member 302. The gel 301 in this example consists of polyvinyl alcohol. This gel 301 contains pure water as an extraction medium.

The supporting member 302 has a supporting main unit 302a with a concave portion, and a brim portion 302b formed in the circumference side of the supporting main unit 302a, wherein the gel 301 is retained in the concave portion of the supporting main unit 302a. An adhesive layer 303 is arranged on the surface of the brim portion 302b, and, in a state before measurement, a release paper 304 to seal the gel 301 retained in the concave portion is stuck to the adhesive layer 303. When the measurement is performed, the release paper 304 is peeled off from the adhesive layer 303 to expose the gel 301 and the adhesive layer 303, and the extraction reservoir 300 is stuck to the skin of the subject through the adhesive layer 303 to fix it in a state where the gel 301 is in contact with the skin of the subject.

[MEASUREMENT PRINCIPLE OF THE PRESENT INVENTION]

Next, the measurement principle of the present invention will be explained, and illustrated based on an exemplary compound which is glucose in the blood. The measurement principle of the present invention will be explained according to the following steps 1 to 3, and an explanation about blood sugar value fluctuation pattern will be given based on measured values (predicted values) concretely obtained in step 4.

[Step 1: Procedure for measurement of area under blood sugar time curve]

<Experiment conditions>

| | |
|---|---|
| Extraction solvent: | Aqueous potassium chloride solution 80 μL |
| Extraction medium | Polyvinyl alcohol gel |
| Extraction form: | Gel chamber |
| Number of extraction sites: | Two sites |
| Extraction area: | 5 mm × 10 mm |
| Extraction time: | For one hour and two hours |
| Number of specimens: | Ten persons |
| Measurement of glucose: | GOD fluorescence absorption spectrometry |
| Measurement of correction parameter: | Ion chromatography |
| Microneedle array shape: | Microneedle length = 300 μm Number of microneedles = 305 |
| Puncture rate: | 6 m/s |
| Measurement of blood sugar: | Forearm SMBG values are measured every 30 minutes. |
| Measurement of blood sugar AUC: | Calculated by trapezoidal approximation from the forearm SMBG values |

<Experiment Procedures>

1) A pretreatment for microhole formation is carried out at the measurement sites (two sites).
2) An extraction reservoir is arranged on each measurement site and extraction of a tissue fluid is started.
3) A fixed amount of sugar is orally loaded.
4) A first extraction reservoir is removed one hour after the start of extraction and an analyte is measured.
5) A second extraction reservoir is removed two hours after the start of extraction and an analyte is measured.

Here, the AUC indicates an area under the blood concentration time curve: AUC.

A correlation between the glucose permeability ($P_{Glc}$) at the puncture site and the sodium ion extraction rate ($J_{Na}$) obtained under the above-mentioned conditions is shown in Fig. 5. Here, the glucose permeability ($P_{Glc}$) is an index showing the glucose permeability at the extraction site, and can be calculated from the following equation (1).

$$P_{Glc} = M_{Glc}/AUC_{BG} \quad (1)$$

$AUC_{BG}$ indicates the area under the blood sugar time curve which is calculated by trapezoidal approximation using the forearm SMBG values. In addition, $M_{Glc}$ is the total amount (mass) of glucose which has been extracted.

Moreover, the sodium ion extraction rate ($J_{Na}$) is an amount of movements per unit time of sodium ion extracted from the measurement site within the measurement time, and is shown by the following equation (2).

$$J_{Na} = M_{Na}/T \quad (2)$$

Here, $M_{Na}$ is the total amount of extracted sodium ions (mole number) and T is an extraction time. Since the subcutaneous concentration of sodium ions is almost constant, the extraction rate of sodium ion becomes an index that reflects only the permeability at the extraction site.

Therefore, the glucose permeability and the sodium ion extraction rate show a good correlation as shown in Fig. 1. Utilizing this correlation, it is possible to precisely predict the glucose permeability at the extraction site by the extraction rate of sodium ion. In other words, if $P_{Glc}$ (calc) is the predicted glucose permeability obtained from the sodium ion extraction rate using an approximate equation in Fig. 5,

$$P_{Glc}\ (calc) = \alpha J_{Na} + \beta \quad (3)$$

(extraction time one hour: $\alpha = 29.003$ $\beta = -1.4862$)

(extraction time two hours): $\alpha = 30.270$ $\beta = -1.2667$)

shall hold.

Then, the equation (1) is changed and the following equation (4) is obtained by substituting $P_{Glc}$ (calc) into $P_{Glc}$. predicted$AUC_{BG} = M_{Glc}/P_{Glc}$(calc)

$$= M_{Glc}/(\alpha J_{Na} + \beta) \quad (4)$$

A correlation between the predicted $AUC_{BG}$ obtained using the equation (4) and the $AUC_{BG}$ obtained from blood sugar value is shown in Fig. 6.

[Step 2: Prediction of 30-Minute Value of Blood Sugar and 90-Minute Value of Blood Sugar Using Area under Blood Sugar Time Curve]

One hour value of the area under the blood sugar time curve ($AUC_{BG}$ (0-60)) is a time integral value at one hour of extraction time. Accordingly, the value obtained by dividing the one hour value of the area under the blood sugar time curve ($AUC_{BG}$ (0-60)) by the extraction time (in this embodiment, it is "1(h)") corresponds to the average blood sugar value ($BG_{average}$ (0 to 1h)) at that time. Therefore, this value is inferred to be correlated to the blood sugar value ($BG_{30min}$) 30 minutes after the start of measurement.

$$AUC_{BG}(0-60)/1=BG_{average}\ (0\ to\ 1h)$$

$$\approx BG_{30min}\ (5)$$

Similarly, the value obtained by dividing the value of the area under the blood sugar time curve at from one hour to two hours after the start of measurement ($AUC_{BG}$(60-120)) by the extraction time (i.e. the value obtained by subtracting the value of the area under the blood sugar time curve one hour after the start of measurement from the value of the area under the blood sugar time curve two hours after the start of measurement) correlates with the blood sugar value 90 minutes after the start of measurement.

$$AUC_{BG}(60-120)/1=BG_{average}\ (1\ to\ 2h)$$

$$\approx BG_{90min}\ (6)$$

The correlation between the one-hour value of the predicted area under the blood sugar time curve (predicted $AUC_{BG}$ (0-60)) obtained from the equation (4) and the 30 minute-value of blood sugar ($BG_{30min}$) is shown in Fig. 7. Also, the correlation between a predicted area under the blood sugar time curve at from one hour to two hours after the start of measurement (predicted $AUC_{BG}$ (60-120)), obtained from the predicted $AUC_{BG}$ (0-60) and the two hour-value of the predicted area under the blood sugar time curve (predicted $AUC_{BG}$(0-120)), and the 90-minute value of blood sugar ($BG_{90min}$) is shown in Fig. 8.

Based on these results, the 30-minute value of blood sugar and the 90-minute value of blood sugar can be determined from the equations (5) and (6) by using the measured values of the area under the blood sugar time curve.

[Step 3: Prediction of 60-Minute Value of Blood Sugar and 90-Minute Value of Blood Sugar Using 0-Minute Value, 30-Minute Value and 60-Minute Value]

This step 3 is a step which characterizes the prediction method of the present invention.

If it was supposed that the change over a period of 0 minute to 60 minutes after the start of measurement was linear, the blood sugar value BG at the time of t is represented like an equation (7.1) using constants A and B. Here, an equation (7.2) and an equation (7.3) are obtained from a 0-minute blood sugar value $BG_{0min}$ obtained by measuring a blood sample; a 30-minute blood sugar value $BG_{30min}$ obtained from a one hour-value of a predicted area under the blood sugar time curve (predicted $AUC_{BG}$ (0-60)); and from the equation (7.1). A 60-minute value of blood sugar $BG_{60min}$ can be obtained from an equation (7.4) expressed by substituting the constants A and B from the two equations, and t = 60 minutes into the equation (7.1).

$$BG = A \times t + B\ (7.1)$$

$$BGS_{0min} = A \times 0 + B\ (7.2)$$

$$BG_{30min} = A \times 30 + B \quad (7.3)$$

$$BG_{60min} = (BG_{30min} - BGS_{0min})/30 \times 60 + BGS_{0min} \quad (7.4)$$

Similarly, if it was supposed that the change over a period of 60 minutes to 120 minutes after the start of measurement was linear, the blood sugar value BG at the time of t is represented like an equation (8.1) using constants C and D. An equation (8.2) and an equation (8.3) are obtained from the 60-minute value of blood sugar value; a 90-minute value of blood sugar value $BG_{90min}$ obtained from a two hour-value of a predicted area under the blood sugar time curve (predicted $AUC_{BG}$ (60 - 120)); and the equation (8.1). C = ($BG_{90min}$ - $BG_{60min}$) / (90 - 60) can be obtained from the equations (8.2) and (8.3). D=$BG_{60min}$ - ($BG_{90min}$ - $BG_{60min}$) / (90 - 60) × 60 can also be obtained. An equation (8.4) is obtained by substituting these values and t = $120_{min}$ into the equation (8.1). A 120-minute value of blood sugar can be hereby predicted.

$$BG = C \times t + D \quad (8.1)$$

$$BG_{60min} = C \times 60 + D \quad (8.2)$$

$$BG_{90min} = C \times 90 + D \quad (8.3)$$

$$BG_{120min} = [(BG_{90min} - BG_{60min})/(90 - 60) \times 120] + BG_{60min} - [(BG_{90min} - BG_{60min})/(90 - 60) \times 60] \quad (8.4)$$

A schematic diagram of such a prediction method is shown in Fig. 9.

$BGS_{0min}$ is measured by collecting blood. $BG_{30min}$ is separately calculated from the liquid which is extracted from the tissue fluid into a reservoir. $BG_{60min}$ is calculated from $BGS_{0min}$ and $BG_{30min}$. This process is explained by the equations (7.1) to (7.4).

In addition, after the predicted calculation of $BG_{60min}$, $BG_{90min}$ is separately calculated from the liquid which is extracted from the tissue fluid into a reservoir. $BG_{120min}$ is calculated from $BG_{60min}$ and $BG_{90min}$. This process is explained by the equations (8.1) to (8.4).

[Step 4: Prediction of Blood Sugar Fluctuation Pattern]

A blood sugar fluctuation pattern is predicted by the method described above using a predicted blood sugar value which has been predicted in 30-minute intervals after the start of measurement. This result is shown in Figures 10 to 12. In Fig. 10, the solid line shows a predicted blood sugar value according to the method of the present invention, and the dashed line shows a blood sugar value in a blood sample collected in 30-minute intervals. Three cases showing a remarkable difference of the blood sugar fluctuation patterns are shown in Figures 10 to 12.

From these results, it it is shown to be possible to distinguish blood sugar fluctuation patterns of A to C shown below (a maximum blood sugar value, a time reaching the maximum blood sugar value, a blood sugar fluctuation rate and the like) according to the method of the present invention.

Fig. 10•••Pattern A: The blood sugar value reaches the maximum value 60 minutes after the start of measurement. The maximum blood sugar value is around 200 mg/dl. After that, the blood sugar value drops at a rate of around 100 mg/dl per hour and becomes around 100 mg/dl 120 minutes after the start of measurement.

Fig. 11•••Pattern B: The blood sugar value reaches the maximum value (in the measurement time) 120 minutes after the start of measurement. The maximum blood sugar value is around 200 mg/dl. The rising rate of the blood sugar value for the first 60 minutes is around 80 mg/dl per hour, and the rising rate of the blood sugar value for the next 60 minutes is around 20 mg/dl per hour. It is possible to predict that pattern A is a slow type of the response rate to insulin secretion, and the like.

Fig. 12•••Pattern C: The blood sugar value reaches the maximum value (in the measurement time) 120 minutes after the start of measurement, but the maximum blood sugar value is around 120 mg/dl, and thus the ups and downs of the blood sugar fluctuations in the measurement time are slight. For patterns A and B, it is possible to infer them to be an excellent type in the action of insulin, and the like.

It is understood that the above-mentioned view obtained from the predicted blood sugar value of each type is corresponding well to the analysis results based on the blood sugar fluctuation obtained from the collected blood measurement.

[Measurement Procedure]

Then, the procedure from puncture to sticking of an extraction reservoir, the measurement of a measurement target in the extraction reservoir, and the concrete calculation will be explained according to two cases (case 1 and case 2).

[Case 1]

Fig. 13 is a flowchart of case 1. This case 1 is a case where the extraction reservoir is freshly replaced on the way of the measurement and then measured.

First of all, an initial value concerning the amount of each of measurement target components, that is, the blood glucose concentration is obtained by drawing blood according to a usual manner (step S0).

In parallel with the step of obtaining this initial value, the tissue fluid is extracted into an extraction reservoir for 60 minutes from the skin where an acceleration treatment has been performed by a puncture. More specifically, the skin 600 of the subject is washed with an alcohol or the like to remove substances (e.g. sweat and dust) that become a disturbance factor of the measurement results (step S1).

After washing has been performed, a microhole 601 is formed on the skin 600 by a puncture instrument 400 (see Fig. 1) where a microneedle chip 500 is attached (step S2). Concretely, a release button 402 is pushed down in a state that an opening (not shown) of the lower part 401a of the puncture instrument 400 is arranged at the site where the microhole 601 of the skin 600 is formed. As a result, fixation of an array chuck 403 is released by a fixation mechanism (not shown), as well as the array chuck 403 moves to the skin 600 side by the force of a spring member 404. And, a microneedle 501 of the microneedle chip 500 (see Fig. 2) attached to the bottom end of the array chuck 403 comes into contact with the subject's skin 600 at a predetermined rate. As a result, the microhole 601 is formed in the epidermis part of the subject's skin 600 as shown in Fig. 3.

Then, as shown in Fig. 14, the subject removes a release paper 304 (see Fig. 4) of a collection member 300 (the first extraction reservoir) and sticks this collection member 300 on the site where the microhole 601 is formed (step 3). As a result, the site at which the microhole 601 is formed and a gel 301 come into contact with each other, as well as the tissue fluid containing glucose and an electrolyte (NaCl) begins to move to the gel 301 through the microhole 601, thereby to start the extraction.

In addition, it is desirable that a timing of an oral sugar loading is immediately before step 0 of obtaining an initial value of blood glucose concentration or immediately after step S3 of sticking the first extraction reservoir, when a sugar tolerance test for evaluating the sugar tolerance and the like in the subject is carried out.

The subject removes the collection member 300 from the skin 600 at a time when a predetermined time has passed (step S4). Because the predetermined time is here set to 60 minutes, extraction of the tissue fluid from the skin is performed continuously over a period of 60 minutes.

Subsequently, in order to extract the tissue fluid into a second extraction reservoir different from the above first extraction reservoir, step S5 (pretreatment of extraction site), step S6 (microhole formation), step S7 (sticking of the second reservoir) and step S8 (removal of the second reservoir) are sequentially carried out in the same manner as in steps S1 to S4.

Moreover, after steps S1 and S2 for the punctuation and alcohol washing have been performed in this Example, each of steps S5 and S6 is carried out, but it is possible to omit each of steps S5 and S6 when the extraction reservoir is replaced (freshly stuck) and the same site is used.

Then, using the first and second extraction reservoirs where the tissue fluid has been extracted, a glucose concentration and a sodium ion concentration are measured (step S9).

As for the glucose concentration and the sodium ion concentration, for example, the extraction reservoir that has finished extraction of analytes from the skin is immersed in a collection liquid (purified water) 31 in a collection tube 30 as shown in Fig. 15, to recover the analytes in the gel 301. The sodium ion concentration and the glucose concentration which are contained in the collection liquid can be measured by the known method. The sodium concentration C (NaCl) in the collection liquid can be measured, for example, by using an ion chromatograph manufactured by Dionex Corporation. In addition, the glucose concentration C (Glc)(ng/ml) in the collection liquid can be measured by a high performance liquid chromatography.

Then, a one-hour value of the predicted area under the blood sugar time curve over a period of from 0 minute to 60 minutes after the start of measurement, and a two-hours value of the predicted area under the blood sugar time curve over a period of from 60 minutes to 120 minutes after the start of measurement are calculated using the equation (4) (step S10).

More concretely, using the sodium concentration C (NaCl) measured in step S9, a sodium ion extraction rate JNa (mmol/h) at the extraction site can be obtained from the following equation:

$$J_{Na} = C(NaCl) \times V(\mu L) \times 10^{-6}/T$$

wherein V is the sum total of the liquid amounts of the gel and the collection liquid, and T is an extraction time in the state as shown in Fig. 14.

Next, using the glucose concentration C (Glc) (ng/ml) measured in step S9, the amount MGlc of glucose extracted is calculated from the following equation:

$$M_{Glc} = C(Glc) \times V(\mu L) \times 10^{-3}$$

Using $J_{Na}$ and $M_{Glc}$ obtained from the above measurements, a predicted $AUC_{BG}$ can be calculated from the equation (4).

Subsequently, using the equations (5) to (8.4), a 30-minute value of blood sugar, a 60-minute value of blood sugar, a 90-minute value of blood sugar, and a 120-minute value of blood sugar are calculated (step S11). And, a blood sugar fluctuation pattern of the subject is obtained by connecting the calculated value and the initial value obtained in step 0 by a line segment (see Figures 10 to 12) (step S12) . As a result, both of the blood sugar fluctuation pattern and the blood sugar AUC are obtained, and they are displayed on a display part.

Further, steps S9 and S10 can be performed by a computer system. This computer system comprises a processor and a memory. The memory is under the control of the processor and includes a software order (a computer program) that is applied to enable the computer system to perform the operations of step S9 and step S10.

A more concrete computer system configuration is shown in Fig. 16. A computer system 900 consists of a personal computer and is composed of a main unit 901, an input part 910 and a display part 920. The main unit 900 has a CPU 901, a ROM 902, a RAM 903, a hard disk 904, a readout device 905, an input/output interface 906, an image output interface 907 and a communication interface 908.

The CPU 901 runs a computer program stored in the ROM 902 and a computer program loaded in the RAM 903. The RAM 903 is used to read out a computer program recorded in the ROM 902 and the hard disk 904. In addition, the RAM 903 is also used as a working area of the CPU 901 when running these computer programs.

Various computer programs to run the operating system, application programs and the like in the CPU 901, and data used for running the computer programs are installed in the hard disk 904. In other words, a program to obtain a blood sugar fluctuation pattern based on the initial value, i.e. the blood glucose concentration, obtained by step 0, and the sodium concentration C (NaCl) and the glucose concentration C (Glc) in the liquid collected from the first extraction reservoir and the second extraction reservoir obtained by step 9 is installed in the hard disk 904. By such installation of this program, steps S10, S11, and S12 are processed. Moreover, a program to display, in the display part 920, the obtained blood sugar fluctuation pattern is included in the hard disk 904.

The readout device 905 is constructed by a CD drive, a DVD drive or the like, and can read out the computer program and data recorded in an external memory such as a recording medium. By this, a program to be executed in the computer system 900 can be updated through the external memory such as a recording medium.

The input part 910 consisting of a mouse and a keyboard is connected to the input/output interface 906. A user can input an initial value, a sodium concentration C (NaCl) and a glucose concentration C (Glc) into the computer system 900 by using the input part 910. In addition, when the initial value, the sodium concentration C (NaCl) and the glucose concentration C (Glc) are measured by various measurement devices, these devices can be connected to the communication interface 908. In this case, the initial value, the sodium concentration C (NaCl) and the glucose concentration C (Glc) can be input into the computer system 900 through the communication interface 908 from the measurement device.

The image output interface 907 is connected to the display part 920 composed of the display or the like and outputs a blood sugar fluctuation pattern, a predicted $AUC_{BG}$ and the like in the display part 920.

In addition, the prediction method according to the present embodiment can also be performed by a device that predicts a concentration fluctuation of each of measurement target components in the blood. For example, the blood sugar fluctuation pattern can be obtained by using the following devices A to C.

<Device A>

A schematic diagram of a device A is shown in Fig. 17. This device A has an introduction part 48, a measurement part 41, an electrode 42 for measuring a glucose concentration, an electrode 43 for measuring a sodium ion concentration, an analysis part 44, a memory part 45, an input part 47, and a display part 46.

First, the initial value as a blood glucose concentration obtained by drawing blood is input from the input part 47. The input initial value is stored in the memory part 45 by the analysis part 44.

Next, the collection liquid collected from the first extraction reservoir with a collection tube 30 shown in Fig. 15 is suctioned with a syringe 32, and retained therein. The collection liquid retained in the syringe 32 is taken out to the introduction part 48 of the device A. The collection liquid moves from the introduction part 48 to the measurement part 41. The electrode 42 for measuring a glucose concentration and the electrode 43 for measuring a sodium ion concentration are arranged in the measurement part 41. The glucose concentration and the sodium ion concentration in the collection liquid are measured by this electrode 42 for measuring a glucose concentration and by this electrode 43 for measuring a sodium ion concentration. The measured glucose concentration and sodium ion concentration are stored in the memory part 45 by the analysis part 44. The analysis part 44 can also obtain a predicted area under the blood sugar time curve from the glucose concentration and the sodium ion concentration by processing step S10. In this case, the glucose concentration, the sodium ion concentration and the predicted area under the blood sugar time curve are stored in the memory part.

Similarly, a glucose concentration and a sodium ion concentration in the collection liquid collected from the second extraction reservoir are measured and stored in the memory part 45 by the analysis part 44. In addition, when the analysis part performs step S10 processing, the glucose concentration, the sodium ion concentration and a predicted area under the blood sugar time curve are stored in the memory part.

The analysis part 44 calls out the initial value, and the glucose concentration and the sodium ion concentration in the collection liquid from the first extraction reservoir and the second extraction reservoir stored in the memory part 45. The analysis part 44 obtains a blood sugar fluctuation pattern and a predicted area under the blood sugar time curve by processing steps S10, 11, and 12 using the initial value, the glucose concentration and the sodium ion concentration. The analysis part 44 outputs the obtained results to the display part 46. Here, in a case where a glucose concentration, a sodium ion concentration and a predicted area under the blood sugar time curve are stored beforehand in the memory part, the analysis part obtains both a blood sugar fluctuation pattern and a predicted area under the blood sugar time curve by processing steps S11 and S12.

In addition, 21 in Fig. 15 is a support member for supporting the gel 301 of the extraction reservoir (collection member) 300.

<Device B>

In this device B, the extraction reservoir (collection member) 300 having the gel 301 which has finished the extraction of the analyte from the skin is set to a special collection cartridge 50 as shown in Fig. 18. This collection cartridge 50 consists of a box-shaped cartridge main unit 51, and an inlet 52 of the collection liquid is formed in one of facing wall surfaces of the cartridge main unit 51, and an outlet 53 of the collection liquid is formed in the other wall surface. The collection member 300 is set to the collection cartridge 50 so that the gel 301 projects to the inside of the cartridge main unit 51 from an opening 54 formed in one side of the cartridge main unit 51.

Then, as shown in Fig. 19, the collection cartridge 50 is set at a predetermined point of the device B. This device B has a tank part 61 and a pump part 62, and a channel of the collection liquid to reach the tank part 61, the pump part 62, the cartridge main unit 51 and the measurement part 63 is formed. In addition, an electrode 64 for measuring a glucose concentration and an electrode 65 for measuring a sodium ion concentration are arranged in the measurement part 63. After setting to the collection cartridge 50, the pump part 62 is driven so that a collection liquid 69 for collecting analytes in the gel is transferred to the inside of the cartridge main unit 51 (see Fig. 19). Though not shown in Figures, a valve is arranged on the downstream side of the outlet 53 of the cartridge main unit 51 and the valve is stopped before transferring the collection liquid 69 to the cartridge main unit 51.

After being left for a certain period of time in a state such that the collection liquid 69 is filled in the cartridge main unit 51, the analytes in the gel 301 are collected in the collection liquid 69. After that, the valve is opened and the pump part 62 is driven to transfer the collection liquid 69 to the measurement part 63, as shown in Fig. 20. Then the glucose concentration and the sodium ion concentration in the transferred collection liquid 69 are measured by the measurement part 63.

Further, the initial value is input from the input part 69. In addition, an analysis part 66 and a memory part 67 in the device B function like the analysis part 44 and the memory part 45 in the device A. As a result, an analysis of a blood sugar fluctuation pattern and a predicted area under the blood sugar time curve is performed using the initial value, the glucose concentration and the sodium ion concentration. The obtained result is output in a display part 68.

<Device C>

A measuring instrument 100 used in this device C includes, as shown in Figures 22 and 23, a display part 1, a memory part 2, an analysis part 3, a power source part 4, an installation part 5 that is a setting part to install a sensor chip 200 and a collection member 300, an electric circuit 6 connected to the sensor chip installed in the installation part 5, an operation button 7 for a user (subject) to operate the device C, a timer part 8, and an input part (not shown).

The installation part 5 has a concave shape with a configuration that enables the sensor chip 200 and the collection member 300 to be installed. The electric circuit 6 includes a circuit 6a for measuring a glucose concentration and a circuit 6b for measuring a sodium ion concentration. The circuit 6a for measuring a glucose concentration includes a terminal 6c and a terminal 6b which are exposed within the installation part 5, and the circuit 6b measuring a sodium ion concentration includes a terminal 6e and a terminal 6f which are exposed within the installation part 5. In addition, the electric circuit 6 includes a switch 6g to switch the circuit 6a for measuring a glucose concentration and the circuit 6b for measuring a sodium ion concentration. The user can operate the switch 6g by operating the operation button 7 and can switch the circuit 6a for measuring a glucose concentration and the circuit 6b for measuring a sodium ion concentration. The operation button 7 is provided to operate the switching of the switch 6g, the switching of the display of the display part 1, the installation of the timer part 8, and the like. The timer part 8 has a function (a function as a time notice means) to notify the user the end time of the extraction so as to finish the extraction within a predetermined period of time after starting the extraction of glucose, and has a built-in alarm device therefor (not shown).

As shown in Figures 24 and 25, the sensor chip 200 is provided with a substrate 201 made of a synthetic resin, a pair of electrodes 202 for measuring a glucose concentration installed on the top surface of the substrate 201, and a pair of electrodes 203 for measuring a sodium ion concentration installed on the top surface of the substrate 201. The electrode 202 for measuring a glucose concentration includes a working electrode 202a wherein a GOD enzyme membrane (GOD: glucose oxidase) is formed at a platinum electrode, and a counter electrode 202b composed of a platinum electrode. On the other hand, the electrode 203 for measuring a sodium ion concentration includes a sodium ion selective electrode 203a composed of silver/silver chloride that has a sodium ion selective membrane, and a silver/silver chloride electrode 203b that is a counter electrode. The working electrode 202a and the counter electrode 202b, which are each the electrode 202 for measuring a glucose concentration, are configured such that they come into contact with the terminals 6c and 6d of the circuit 6a for measuring a glucose concentration, respectively, in a state that the sensor chip 200 is installed in the installation part 5 of the device C. Similarly, the sodium ion selective electrode 203a and the silver/silver chloride electrode 203b, which are each the electrode 203 for measuring a sodium ion concentration, are configured such that they come into contact with the terminals 6e and 6f of the circuit 6b for measuring a sodium ion concentration, respectively, in the state that the sensor chip 200 is installed in the installation part 5 of the device C.

At the time of the measurement, a constant voltage is applied between the working electrode and the counter electrode which are each an electrode for measuring a glucose concentration, through a constant voltage control circuit, thereby to obtain a generated electric current value $I_{Glc}$ (see Figures 24 to 25). A glucose concentration is obtained by substituting this electric current value $I_{Glc}$ to an equation:

$$(C(Glc) = A \times I_{Glc} + B)$$

Then, the circuit is switched by the operation button and a constant electric current is applied between the electrodes for measuring a sodium ion concentration through a constant voltage control circuit, and a sodium ion concentration is obtained from the resulting voltage value $V_{Na}$ using an equation:

$$(C(NaCl) A' \times V_{Na} + B').$$

The initial value is input in the input part. In addition, the analysis part 3 and the memory part 2 in the device C function like the analysis part 44 and the memory part 45 in the device A. As a result, an analysis of a blood sugar fluctuation pattern and a predicted area under the blood sugar time curve is performed using the initial value, the glucose concentration and the sodium ion concentration. The obtained result is output in the display part 1.

[Case 2]

Fig. 28 is a flowchart of case 2. This case 2 is a case where the two extraction reservoirs are stuck on the skin and one extraction reservoir is removed on the way after the passage of a certain period of time.

Even in case 2, first of all, an initial value concerning the amount of each of measurement target components, that is, the blood glucose concentration is obtained by drawing blood according to a usual manner (step S20).

In parallel with the step of obtaining this initial value, the tissue fluid is extracted into an extraction reservoir for 60 minutes or 120 minutes from the skin where an acceleration treatment has been performed by a puncture. More specifically, the skin 600 of the subject is washed with an alcohol or the like to remove substances (e.g. sweat and dust) that become a disturbance factor of the measurement results (step S21).

After washing has been performed, two microholes 601 are formed on the skin 600 by a puncture instrument 400 (see Fig. 1) where a microneedle chip 500 is attached (step S22).

Then, as shown in Fig. 14, the subject removes release papers 304 (see Fig. 4) of two collection members 300 respectively and sticks each collection member 300 on the two sites where the microholes 601 are formed (step 23). As a result, the site where the microhole 601 is formed and the gel 301 come into contact with each other, as well as the tissue fluid containing glucose and an electrolyte (NaCl) begins to move to the gel 301 through the microhole 601, and extraction is hereby started.

The subject removes one of the collection members 300 from the skin 600 at the time when a predetermined time has passed (step S24). Because the predetermined time is here set to 60 minutes, extraction of the tissue fluid from the skin is performed continuously over a period of 60 minutes.

Then, the subject removes the remaining other collection member 300 from the skin 600 at the time when another predetermined time has passed (step S25). Because another predetermined time is here set to 60 minutes, extraction of the tissue fluid from the skin is performed continuously in the other collection member 300 over a period of 120 minutes.

Subsequently, the glucose concentration and the sodium ion concentration are measured using the two extraction reservoirs wherein the tissue liquid has been extracted (step S26).

Then, using an equation (4), a one-hour value of a predicted area under the blood sugar time curve over a period of from 0 minute to 60 minutes after the start of measurement and a two-hour value of a predicted area under the blood sugar time curve over a period of from 60 minutes to 120 minutes after the start of measurement are calculated (step 27). With this case 2, the two-hour value of a predicted area under the blood sugar time curve over a period of from 60 minutes to 120 minutes after the start of measurement can be calculated by subtracting a predicted blood sugar AUC obtained in one of the collection members 300 from a predicted blood sugar AUC obtained in the other collection member 300.

Subsequently, a 30-minute value of blood sugar, a 60-minute value of blood sugar, a 90-minute value of blood sugar, and a 120-minute value of blood sugar are calculated (step S28) using the equations (5) to (8.4), and a blood sugar fluctuation pattern (see Figures 10 to 12) of the subject is obtained by connecting the calculated values with a line segment (step S29) . As a result, both of the blood sugar fluctuation pattern and the blood sugar AUC are obtained, and they are displayed on a display part.

In addition, it is possible to appropriately apply the computer system described above and the device described above to obtain the blood sugar fluctuation pattern to the case 2. More specifically, the computer system and the device may be set up so that the two-hour value of a predicted area under the blood sugar time curve over a period of from 60 minutes to 120 minutes after the start of measurement is calculated by subtracting a predicted blood sugar AUC obtained in one collection member 300 from a predicted blood sugar AUC obtained in the other collection member 300.

In addition, an example of measuring the glucose amount in the tissue liquid is described in the above-mentioned embodiment, but the present invention is not limited thereto, and may be used as some kinds of indexes by measuring the amounts of substances other than glucose contained in the tissue liquid. The substances measured by the present invention include, for example, a biochemical component and a medicament administered to a subject. Examples of the biochemical components include a protein which is one of biochemical components, such as albumin, globulin, enzymes and the like. Examples of the biochemical components other than proteins include creatinine, creatine, uric acid, amino acids, fructose, galactose, pentose, glycogen, lactic acid, pyruvic acid and a ketone body. Further, the medicament includes digitalis preparations, theophyllines, antiarrhythmic agents, antiepileptic agents, aminoglycoside antibiotics, glycopeptide antibiotics, antithrombotic agents and immunosuppressants.

In addition, in the embodiment described above, an example of the calculated and predicted blood sugar AUC and the concentration fluctuation are, as they are, displayed in the display part, but the present invention is not limited thereto, and a value obtained by dividing the calculated and predicted blood sugar AUC by the extraction time may also be displayed in the display part. As a result, since a predicted blood sugar AUC per unit time can be obtained, those values can be easily compared even in a case where the extraction time is different.

Moreover, in the embodiment described above, an analysis of blood sugar fluctuation pattern by the extraction of the tissue liquid is performed during the period of the first extraction time and the second extraction time (in case 1, both extraction times are 60 minutes, and, in case 2, the first extraction time is 60 minutes and the second extraction time is 120 minutes), but it is possible to extract the tissue fluid during a further period of time (e.g., the third period). In this case, an extraction reservoir may be freshly stuck every extraction period, or extraction reservoirs only of the number of the extraction periods may be stuck first and then peeled off sequentially after the passage of the predetermined time.

## Claims

**1.** A method for predicting a concentration fluctuation of a measurement target component in the blood, comprising steps of:

obtaining an initial value of the amount relating to the measurement target component in a subject;
obtaining, as a first measurement value, the value of the area under the blood concentration time curve of the measurement target component, from the amount of the measurement target component contained in a tissue liquid extracted during a first extraction period from a microhole formed on the skin of the subject;
obtaining, as a second measurement value, the value of the area under the blood concentration time curve of the measurement target component, from the amount of the measurement target component contained in a tissue liquid extracted during a second extraction period from the microhole formed on the skin of the subject; and
predicting the concentration fluctuation of the measurement target component in the blood from the initial value, the first measurement value and the second measurement value.

**2.** The method according to claim 1, wherein the second extraction period is a period after the completion of the first extraction period.

**3.** The method according to claim 2, wherein the prediction step comprises:

obtaining a first blood concentration of the measurement target component at a first point from the first measurement value;
obtaining a second blood concentration of the measurement target component at a second point from the second measurement value; and
predicting the concentration fluctuation of the measurement target component in the blood from the initial value, the first blood concentration and the second blood concentration.

**4.** The method according to claim 1, wherein the first extraction period and the second extraction period are started at the same time, and the second extraction period is longer than the first extraction period.

**5.** The method according to claim 4, wherein the prediction step comprises:

obtaining a first blood concentration of the measurement target component at a first point from the first measurement value;
obtaining a second blood concentration of the measurement target component at a second point from the difference between the second measurement value and the first measurement value; and
predicting the concentration fluctuation of the measurement target component in the blood from the initial value, the first blood concentration and the second blood concentration.

**6.** The method according to any one of claims 1 to 5, wherein the measurement target component is glucose, and the concentration fluctuation of the measurement target component is a fluctuation of a blood glucose value.

**7.** The method according to claim 6, wherein the fluctuation of a blood sugar value is expressed by each parameter of a prediction of the maximum blood sugar value, a prediction of the time reaching the maximum blood sugar value, and a blood sugar value fluctuation rate.

**8.** The method according to any one of claims 1 to 7,
wherein

the first measurement value is a value corrected by the concentration of sodium ion contained in the tissue liquid extracted during the first extraction period, and
the second measurement value is a value corrected by the concentration of sodium ion contained in the tissue liquid extracted during the second extraction period.

**9.** The method according to any one of claims 1 to 8, wherein at least one of the first extraction period and the second extraction period is 60 minutes or more.

**10.** The method according to any one of claims 1 to 9, wherein the sum of the first extraction period and the second extraction period is 120 minutes or more.

**11.** The method according to any one of claims 1 to 9, wherein the second extraction period is 120 minutes or more.

**12.** The method according to any one of claims 1 to 11, wherein the initial value is the blood concentration value of the measurement target component obtained by drawing blood from the subject.

**13.** A device for predicting a concentration fluctuation of a measurement target component in the blood, comprising:

an input part for inputting an initial value relating to the amount of a measurement target component of a subject;
a first collection member for retaining a tissue liquid extracted during a first extraction period from a microhole formed on the skin of the subject;
a second collection member for retaining a tissue liquid extracted during a second extraction period from the microhole formed on the skin of the subject;
a measurement part for measuring the amount of the measurement target component contained in the first collection member and the amount of the measurement target component contained in the second collection member; and
an analysis part configured for performing operations, comprising:

obtaining, as a first measurement value, the value of the area under the blood concentration time curve of the measurement target component during the first extraction period, from the amount of the measurement target component contained in the first collection member;
obtaining, as a second measurement value, the value of the area under the blood concentration time curve of the measurement target component during the second extraction period, from the amount of the measurement target component contained in the second collection member; and
predicting the concentration fluctuation of the measurement target component in the blood from the initial value, the first measurement value and the second measurement value.

**14.** The device according to claim 13, wherein the second extraction period is a period after the completion of the first extraction period.

**15.** The device according to claim 14, wherein the analysis part is configured for performing further operations, comprising:

obtaining a first blood concentration of a measurement target component at a first point from the first measurement value; and
obtaining a second blood concentration of a measurement target component at a second point from the second measurement value; wherein the concentration fluctuation of the measurement target component in the blood is predicted from the initial value, the first blood concentration and the second blood concentration.

**16.** The device according to claim 13, wherein the first extraction period and the second extraction period are started at the same time, and the second extraction period is longer than the first extraction period.

**17.** The device according to claim 16, wherein the analysis part is configured for performing further operations, comprising:

obtaining a first blood concentration of a measurement target component at a first point from the first measurement value; and
obtaining a second blood concentration of a measurement target component at a second point from a value obtained by subtracting the first measurement value from the second measurement value; wherein the concentration fluctuation of the measurement target component in the blood is predicted from the initial value, the first blood concentration and the second blood concentration.

**18.** The device according to any one of claims 13 to 17, wherein the measurement target component is glucose and the concentration fluctuation of the measurement target component is a fluctuation of blood glucose value.

FIG. 1

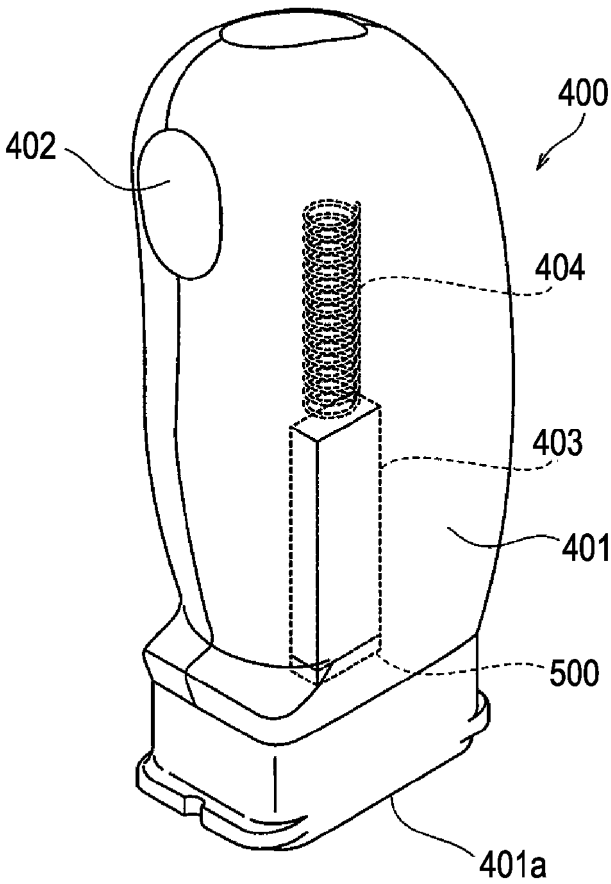


FIG. 2

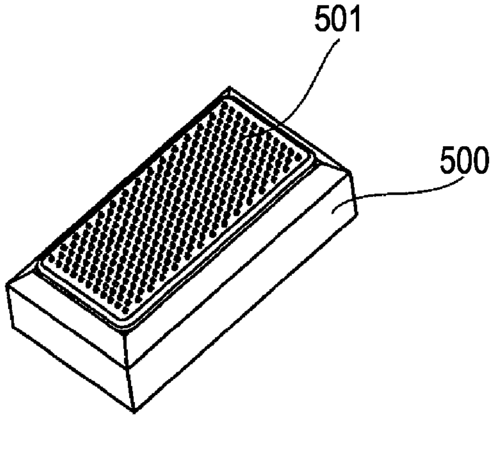

*FIG. 3*

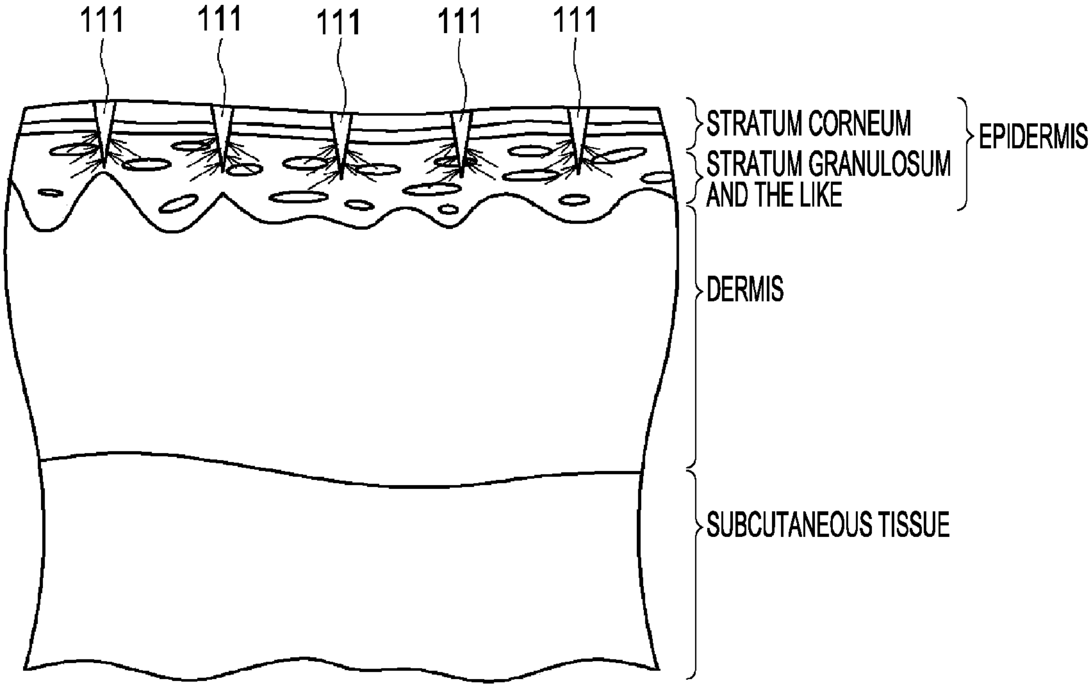
111
111
111
111
111
STRATUM CORNEUM
STRATUM GRANULOSUM AND THE LIKE
EPIDERMIS
DERMIS
SUBCUTANEOUS TISSUE

*FIG. 4*

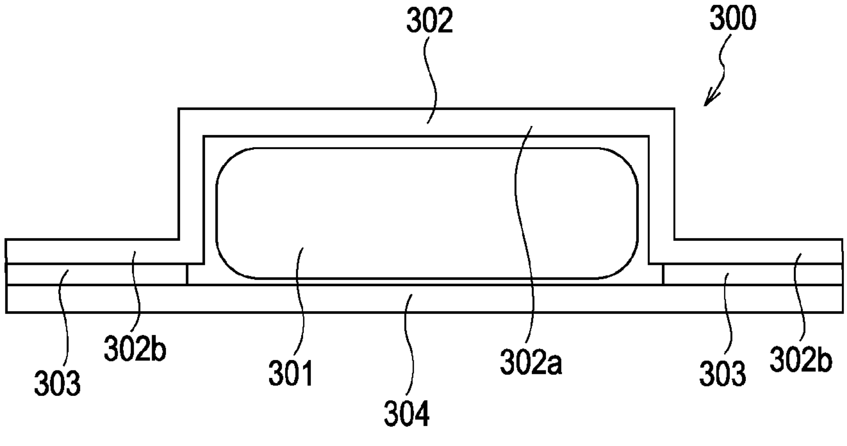
302
300
303
302b
301
304
302a
303
302b

FIG. 5

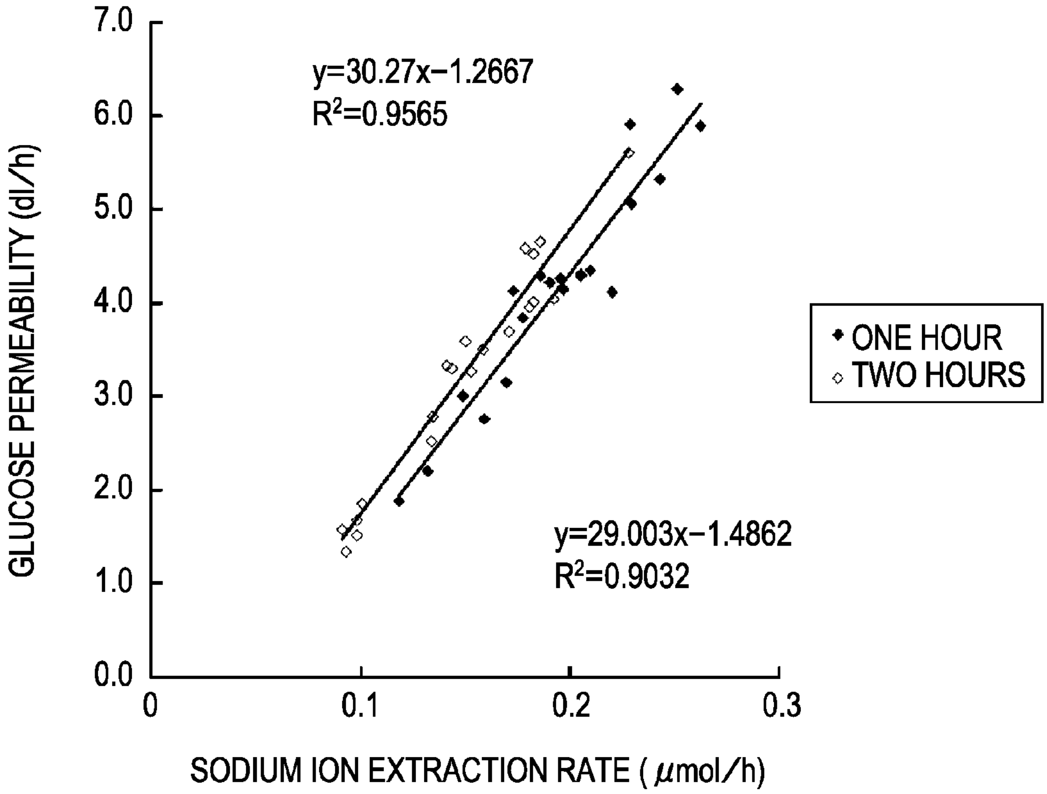

y=30.27x−1.2667
R2=0.9565
y=29.003x−1.4862
R2=0.9032
GLUCOSE PERMEABILITY (dl/h)
7.0
6.0
5.0
4.0
3.0
2.0
1.0
0.0
0
0.1
0.2
0.3
SODIUM ION EXTRACTION RATE (μmol/h)
ONE HOUR
TWO HOURS

FIG. 6

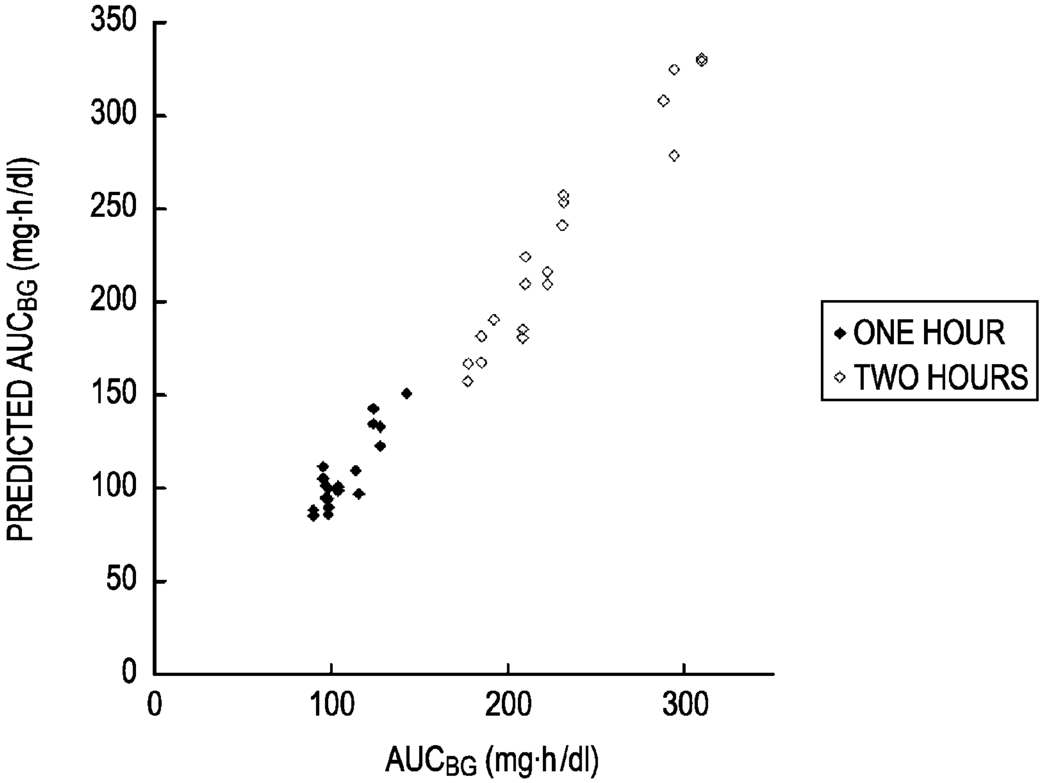
PREDICTED AUC$_{BG}$ (mg·h/dl)
350
300
250
200
150
100
50
0
0
100
200
300
AUC$_{BG}$ (mg·h/dl)
ONE HOUR
TWO HOURS

FIG. 7

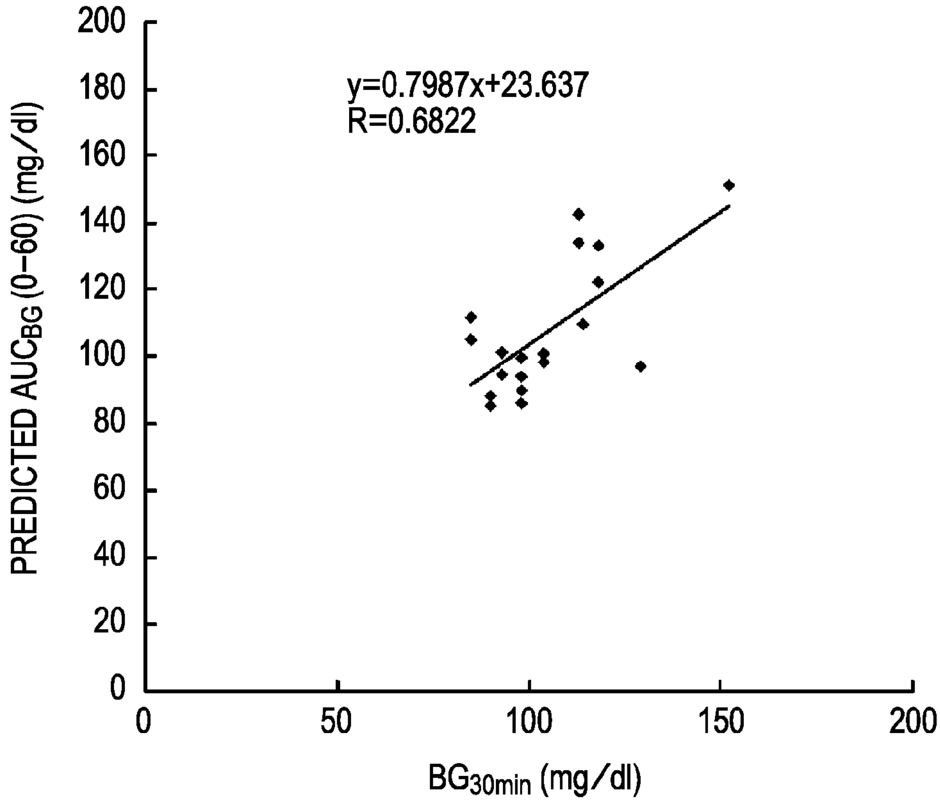
y=0.7987x+23.637
R=0.6822
PREDICTED AUC$_{BG}$ (0-60) (mg/dl)
200
180
160
140
120
100
80
60
40
20
0
0
50
100
150
200
BG$_{30min}$ (mg/dl)

FIG. 8

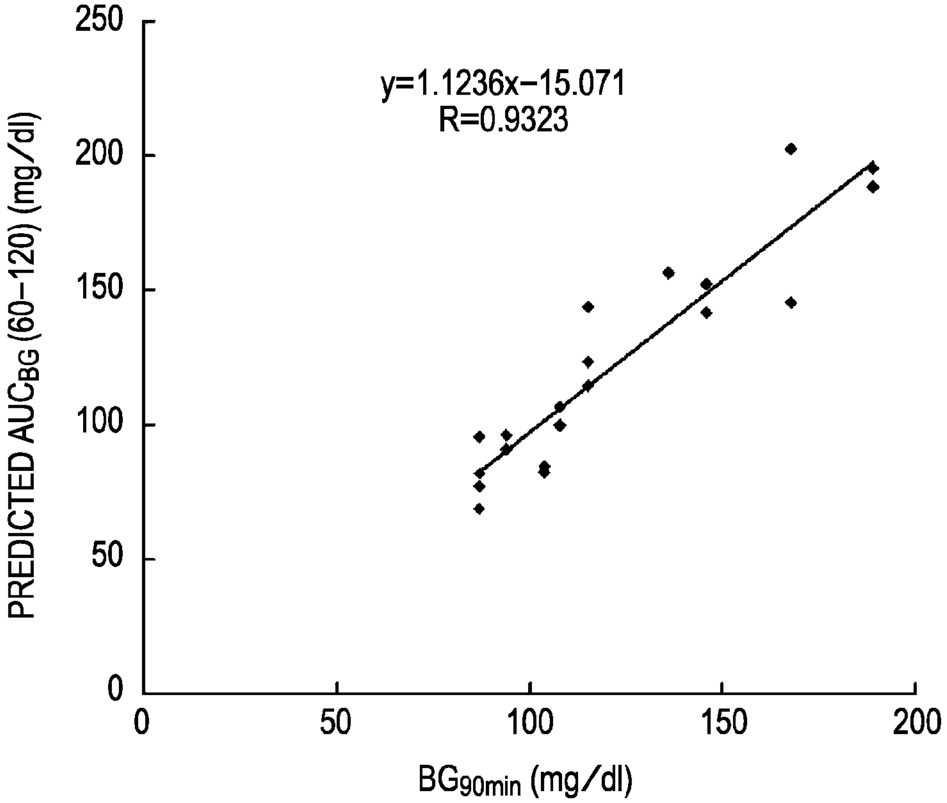
y=1.1236x−15.071
R=0.9323
PREDICTED AUCBG (60−120) (mg/dl)
250
200
150
100
50
0
0
50
100
150
200
BG90min (mg/dl)

FIG. 9

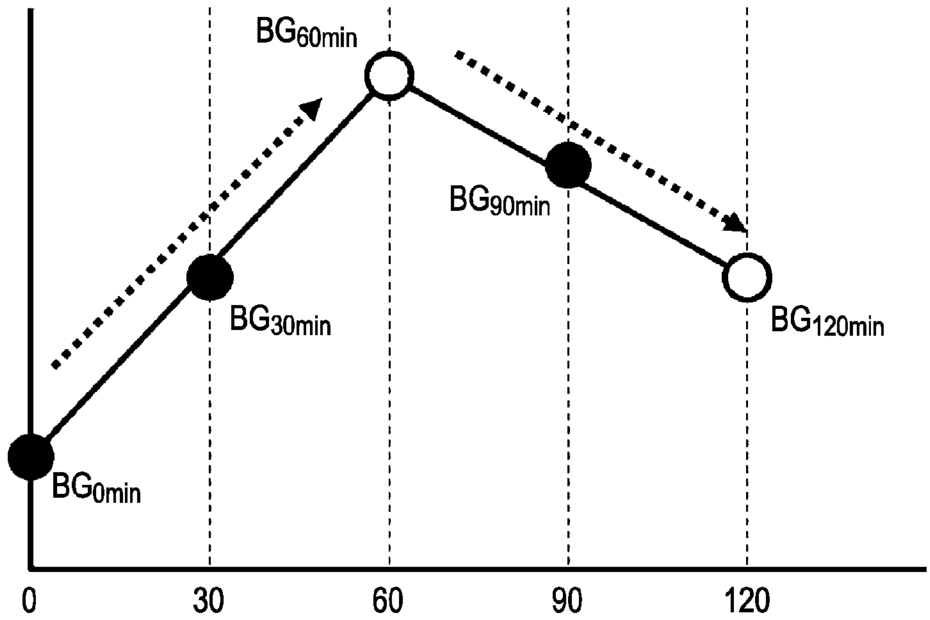

FIG. 10

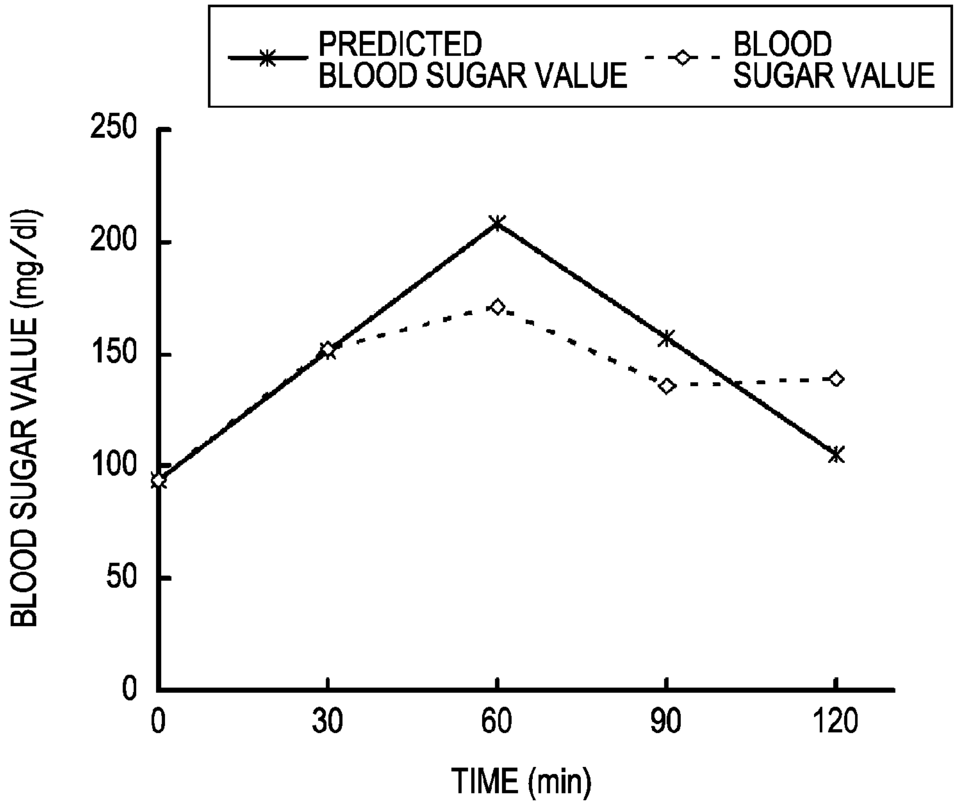
PREDICTED BLOOD SUGAR VALUE
BLOOD SUGAR VALUE
250
200
150
100
50
0
BLOOD SUGAR VALUE (mg/dl)
0
30
60
90
120
TIME (min)

FIG. 11

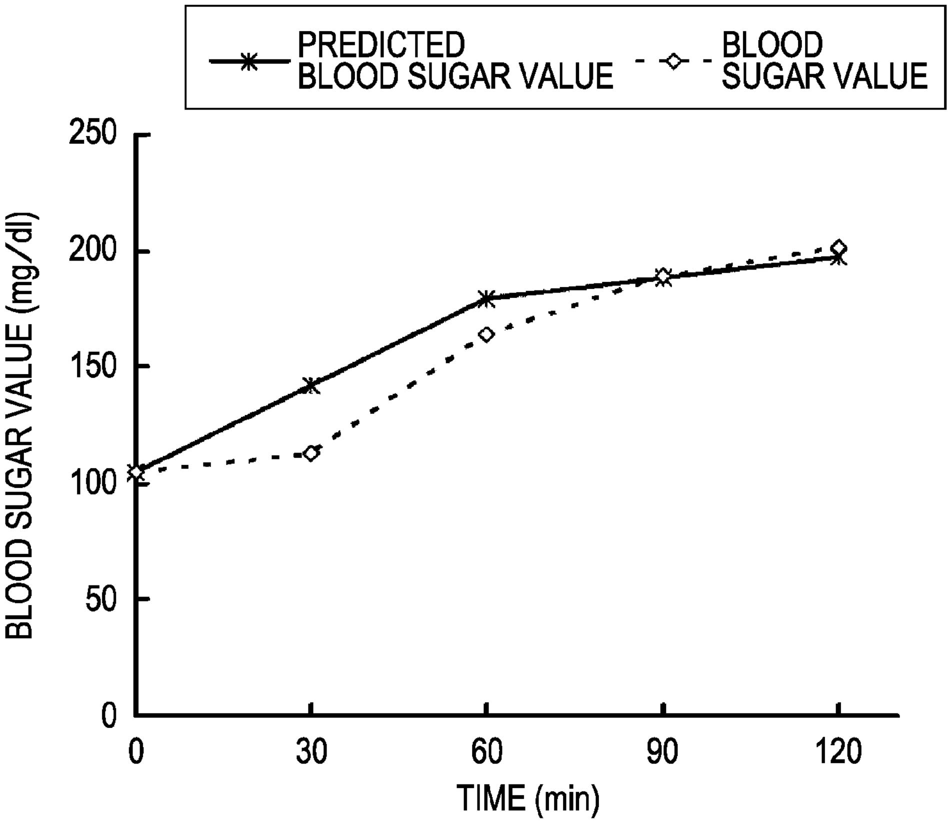
PREDICTED
BLOOD SUGAR VALUE
BLOOD
SUGAR VALUE
BLOOD SUGAR VALUE (mg/dl)
250
200
150
100
50
0
0
30
60
90
120
TIME (min)

FIG. 12

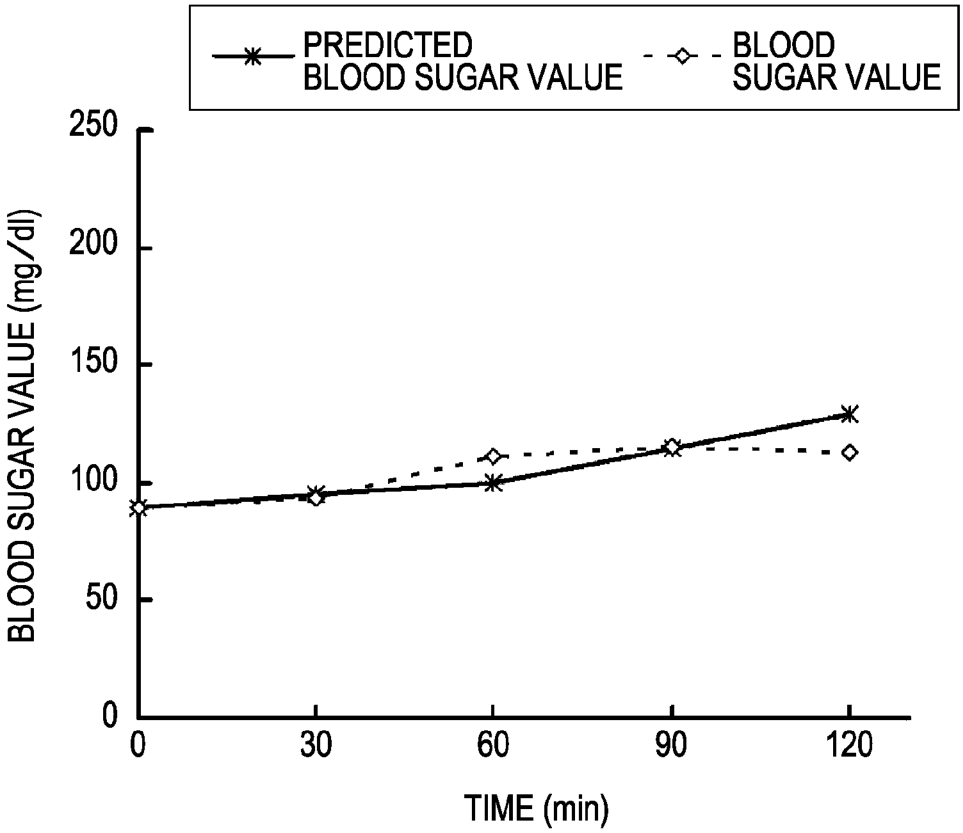
PREDICTED
BLOOD SUGAR VALUE
BLOOD
SUGAR VALUE
BLOOD SUGAR VALUE (mg/dl)
250
200
150
100
50
0
0
30
60
90
120
TIME (min)

FIG. 13

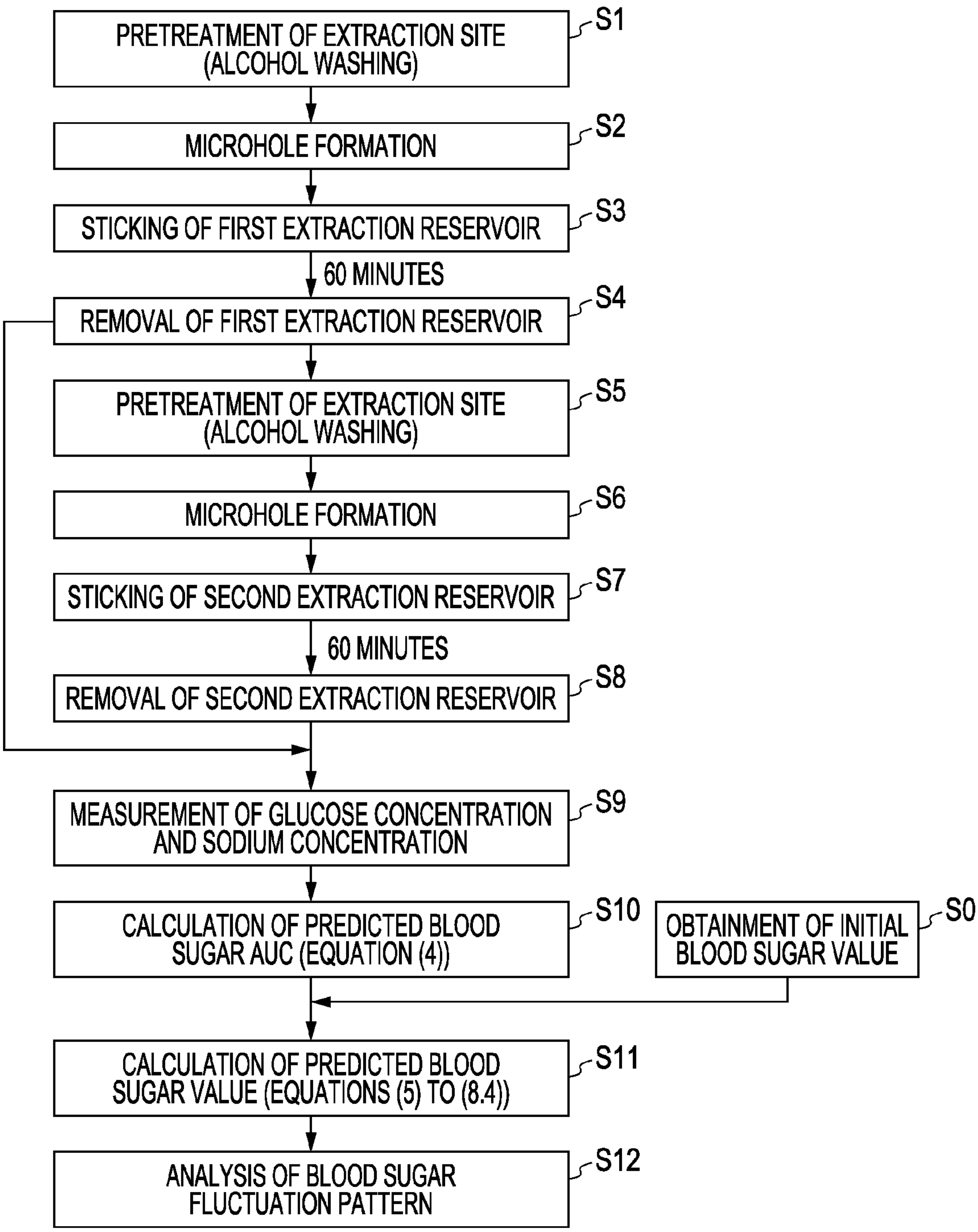
PRETREATMENT OF EXTRACTION SITE (ALCOHOL WASHING)
S1
MICROHOLE FORMATION
S2
STICKING OF FIRST EXTRACTION RESERVOIR
S3
60 MINUTES
REMOVAL OF FIRST EXTRACTION RESERVOIR
S4
PRETREATMENT OF EXTRACTION SITE (ALCOHOL WASHING)
S5
MICROHOLE FORMATION
S6
STICKING OF SECOND EXTRACTION RESERVOIR
S7
60 MINUTES
REMOVAL OF SECOND EXTRACTION RESERVOIR
S8
MEASUREMENT OF GLUCOSE CONCENTRATION AND SODIUM CONCENTRATION
S9
CALCULATION OF PREDICTED BLOOD SUGAR AUC (EQUATION (4))
S10
OBTAINMENT OF INITIAL BLOOD SUGAR VALUE
S0
CALCULATION OF PREDICTED BLOOD SUGAR VALUE (EQUATIONS (5) TO (8.4))
S11
ANALYSIS OF BLOOD SUGAR FLUCTUATION PATTERN
S12

FIG. 14

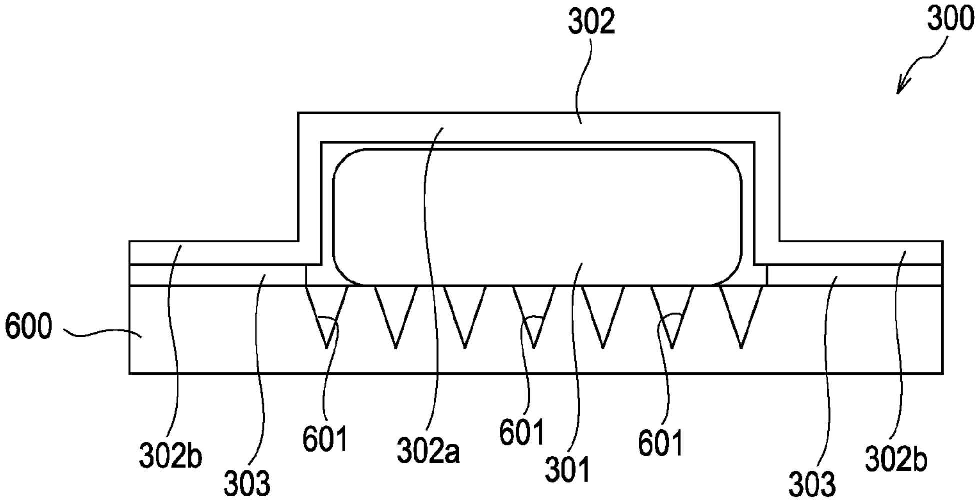
302
300
600
302b
303
601
302a
601
301
601
303
302b

FIG. 15

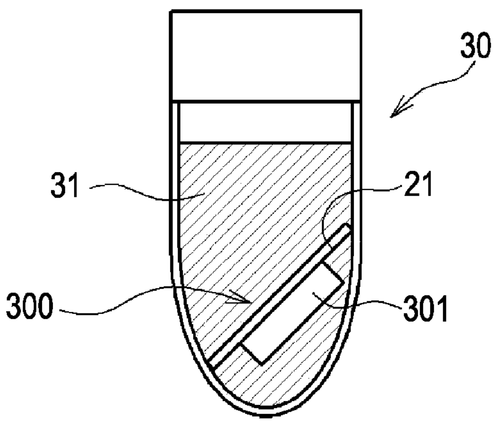
30
31
21
300
301

FIG. 16

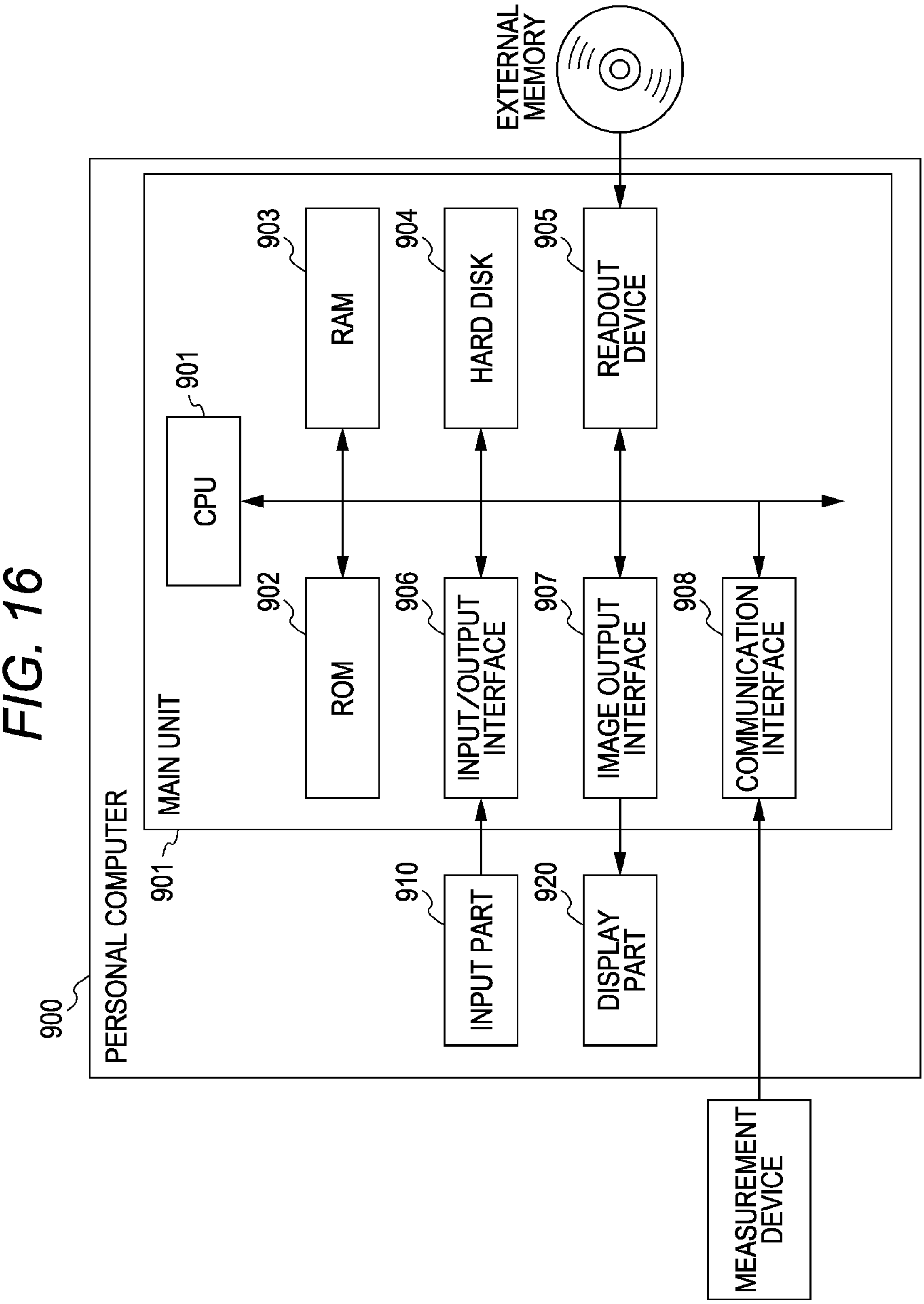
900
PERSONAL COMPUTER
901
MAIN UNIT
901
CPU
902
ROM
903
RAM
904
HARD DISK
905
READOUT DEVICE
906
INPUT/OUTPUT INTERFACE
907
IMAGE OUTPUT INTERFACE
908
COMMUNICATION INTERFACE
910
INPUT PART
920
DISPLAY PART
EXTERNAL MEMORY
MEASUREMENT DEVICE

FIG. 17

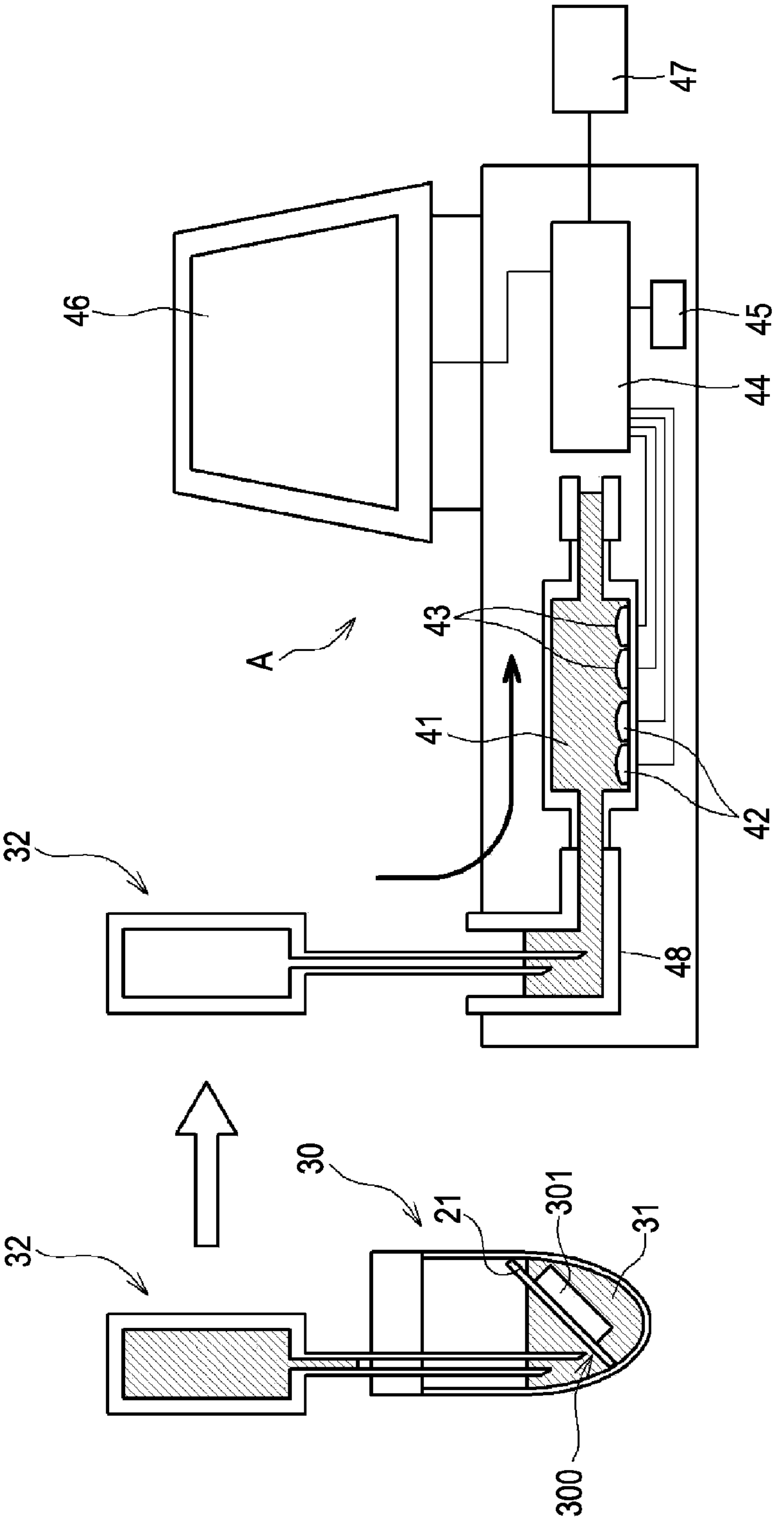

FIG. 18

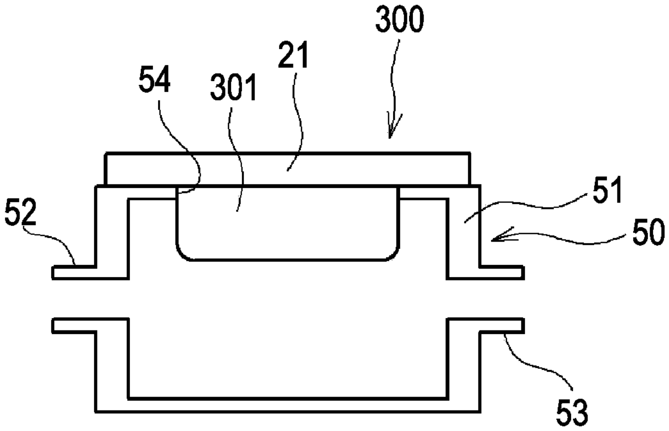

FIG. 19

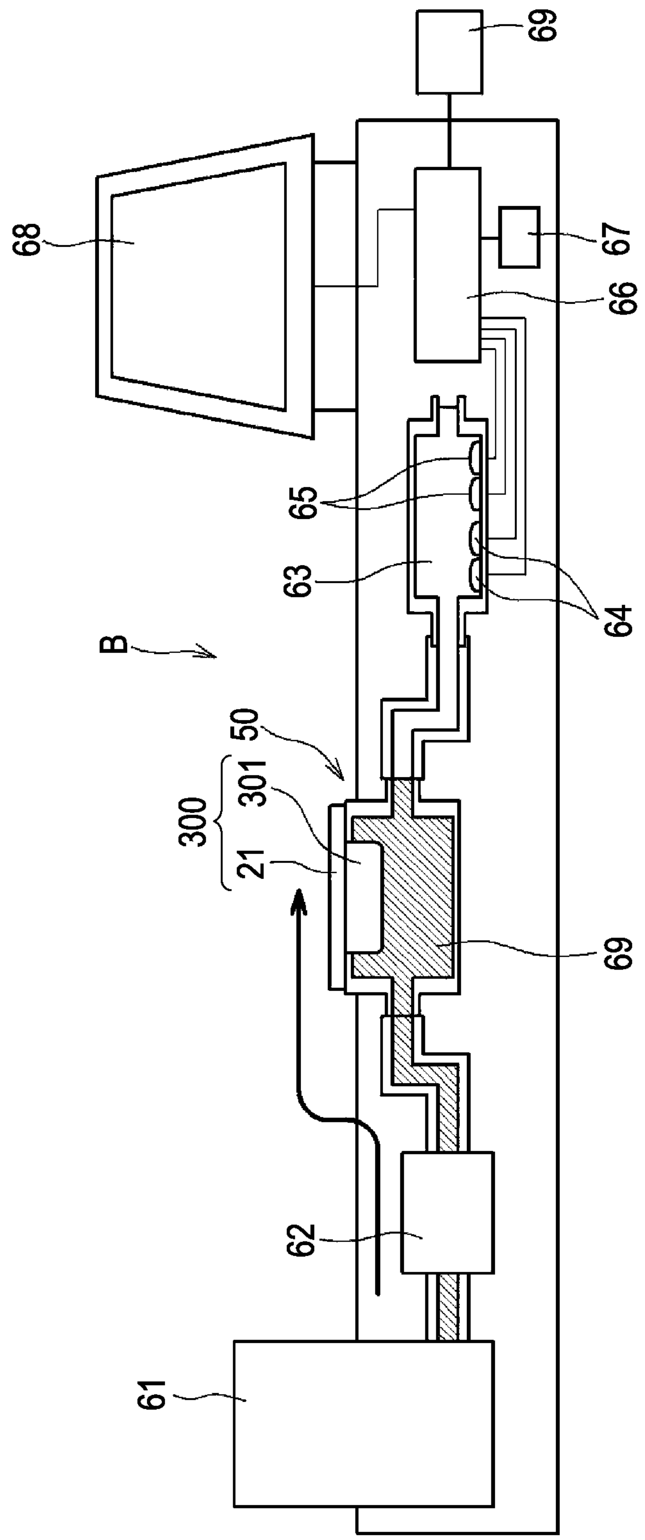

FIG. 20

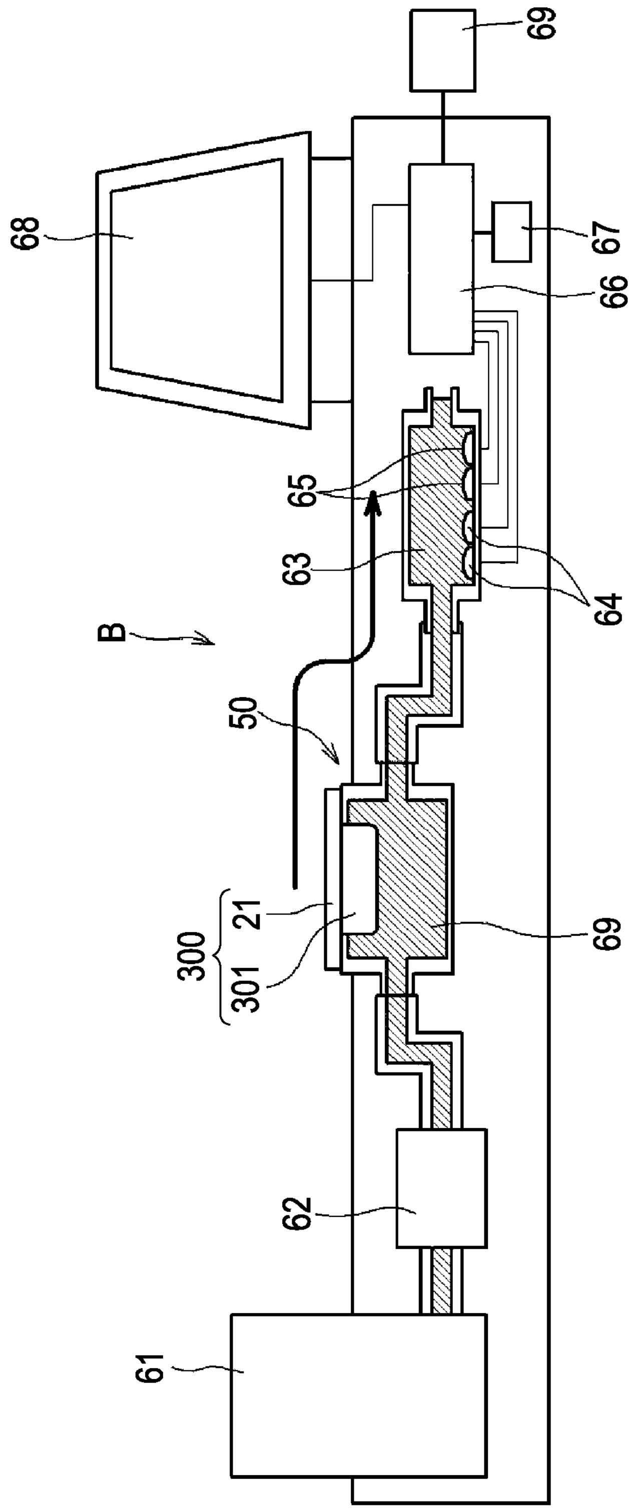

FIG. 21

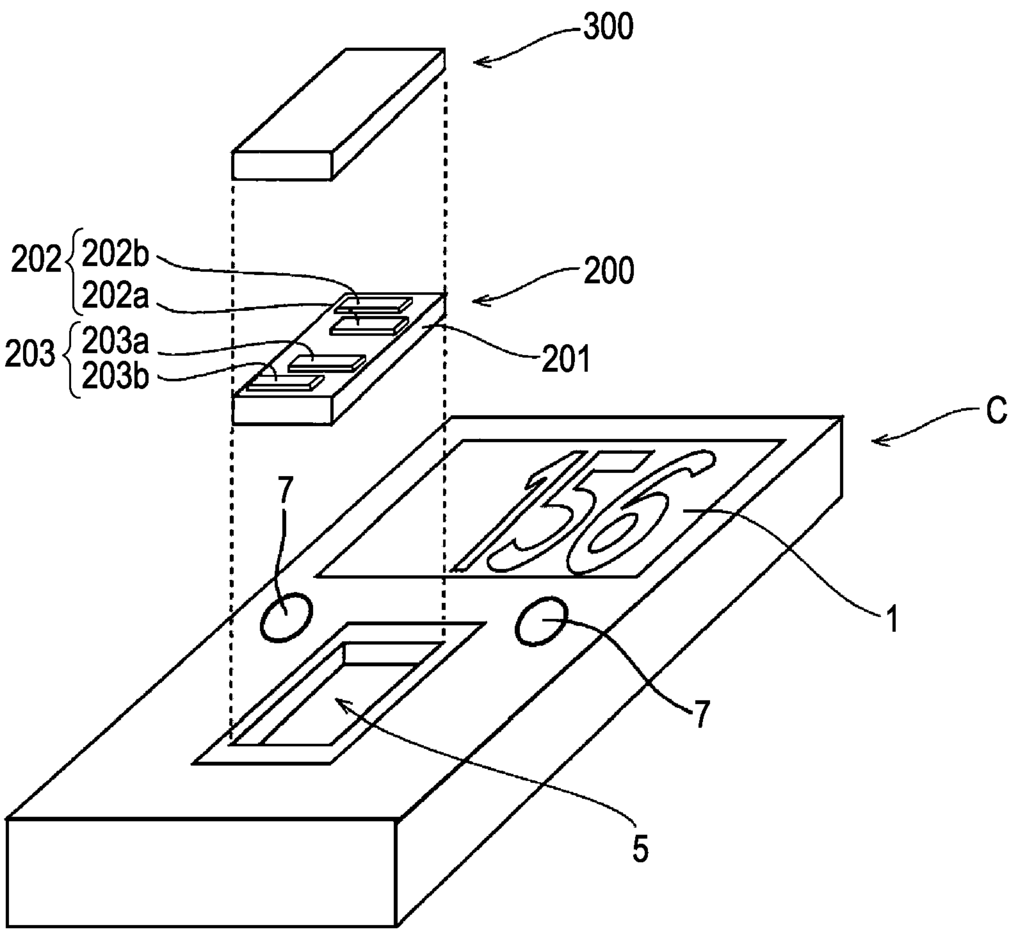

FIG. 22

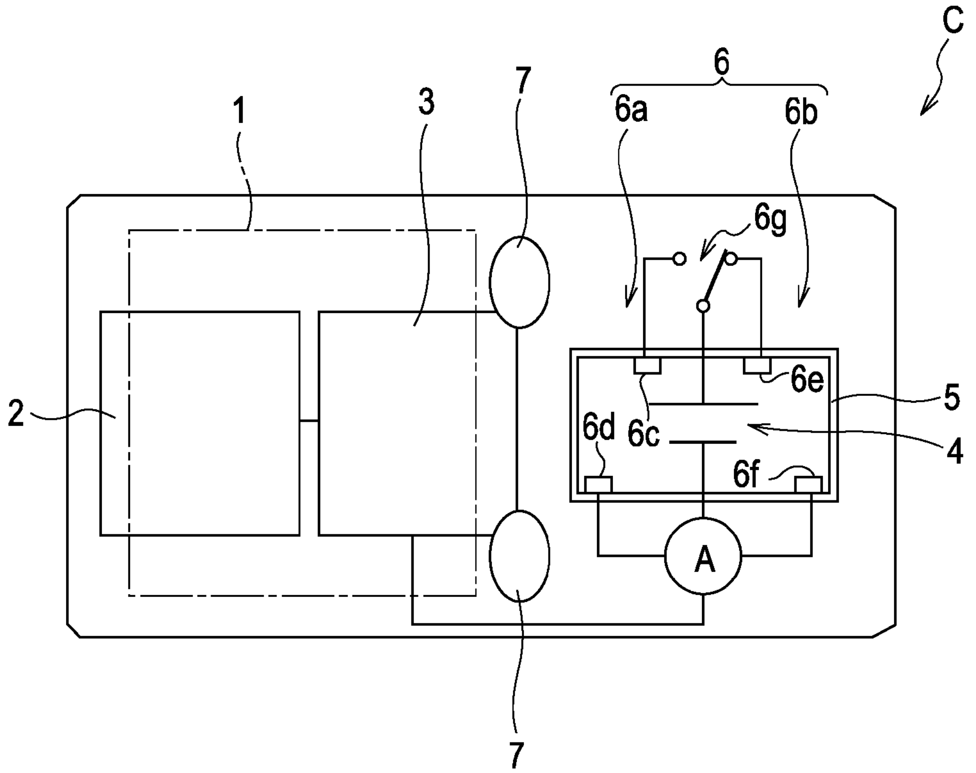
C
6
6a
6b
1
3
7
6g
2
6e
5
6d
6c
4
6f
A
7

FIG. 23

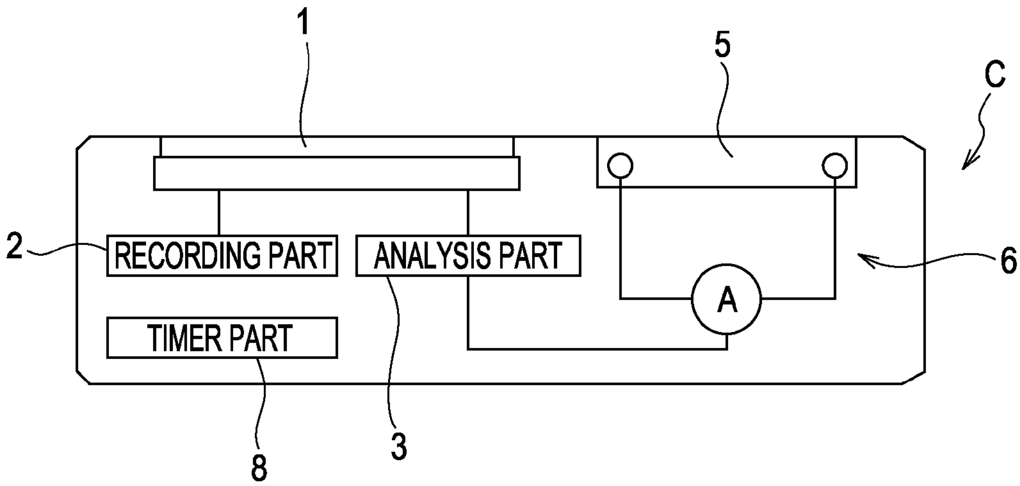
1
5
C
2
RECORDING PART
ANALYSIS PART
6
A
TIMER PART
8
3

FIG. 24

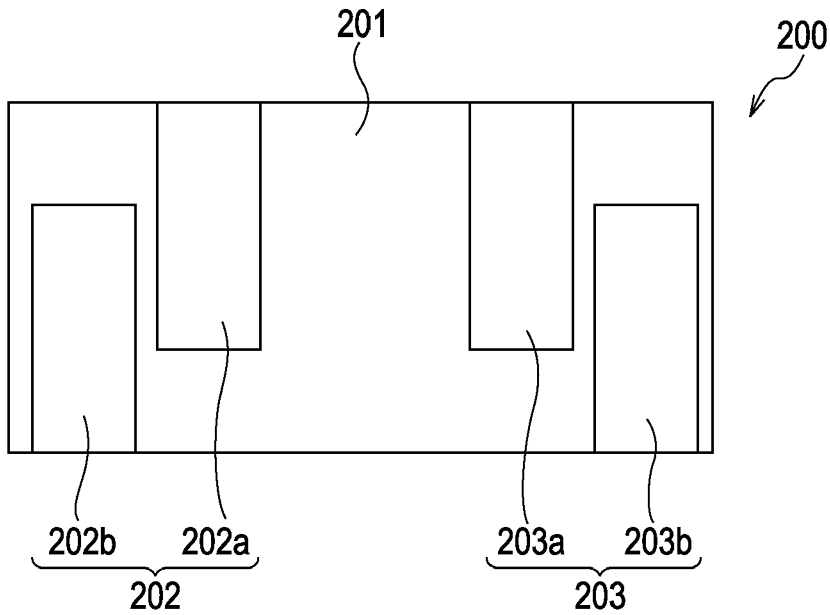
201
200
202b
202a
203a
203b
202
203

FIG. 25

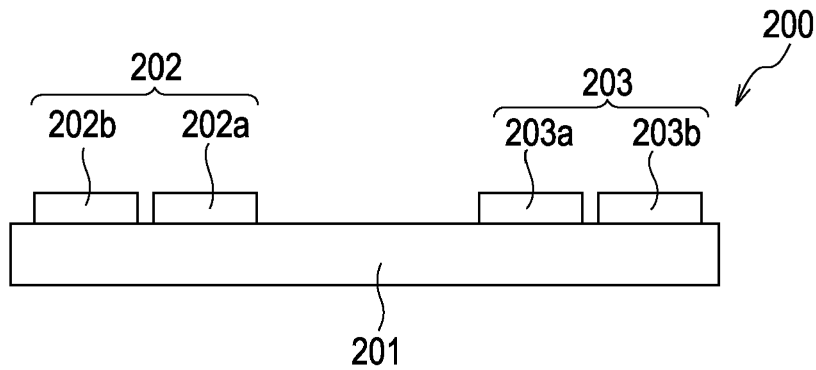
200
202
203
202b
202a
203a
203b
201

FIG. 26

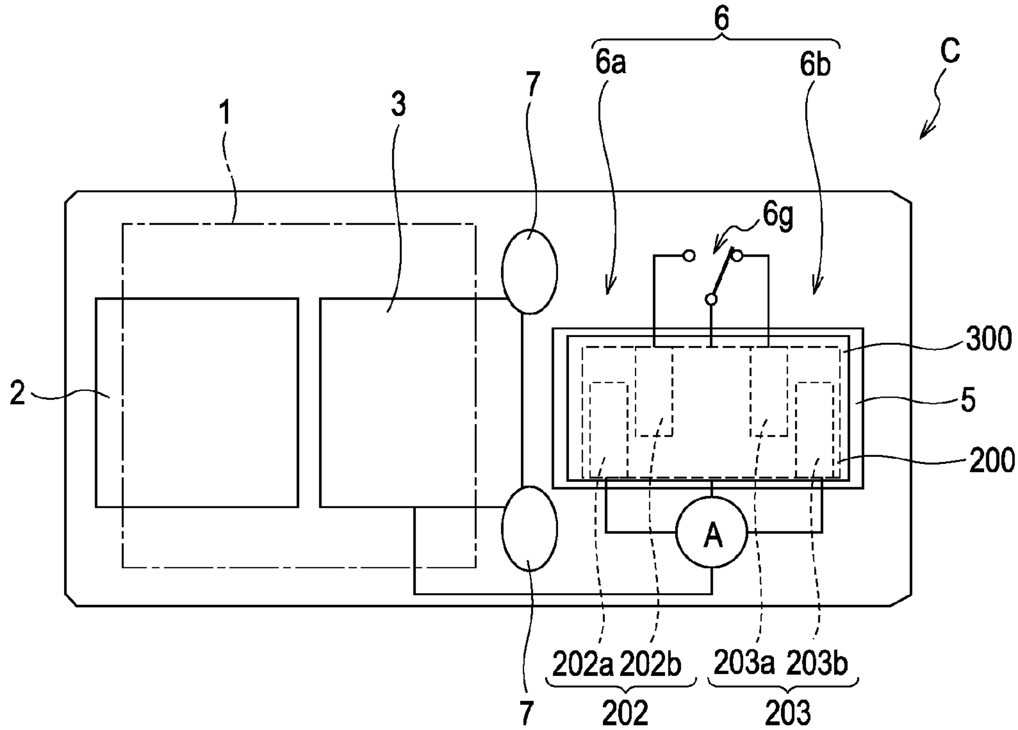
6
6a
6b
C
7
1
3
6g
300
2
5
200
A
202a 202b
203a 203b
7
202
203

FIG. 27

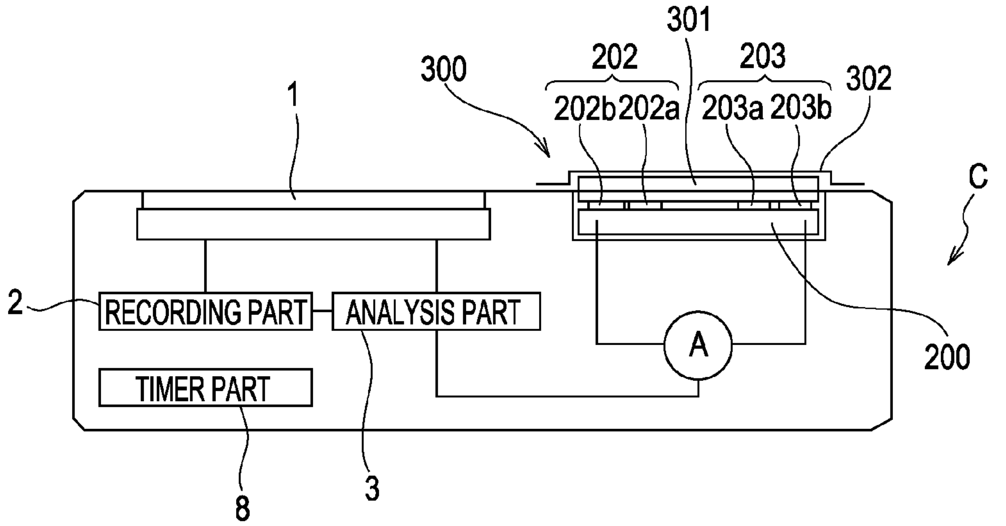
301
202
203
300
302
1
202b 202a
203a 203b
C
2
RECORDING PART
ANALYSIS PART
A
200
TIMER PART
8
3

FIG. 28

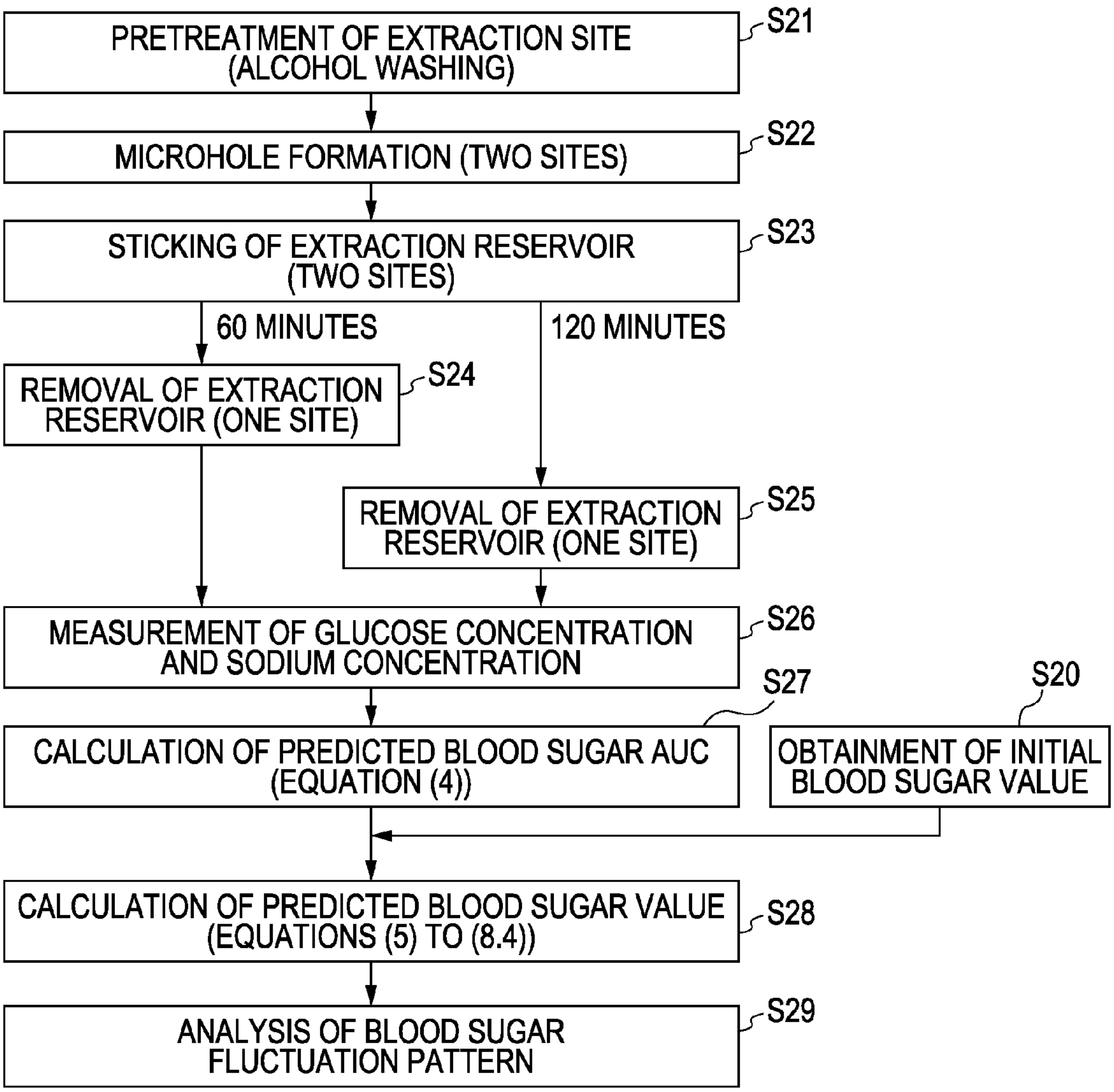
PRETREATMENT OF EXTRACTION SITE (ALCOHOL WASHING)
S21
MICROHOLE FORMATION (TWO SITES)
S22
STICKING OF EXTRACTION RESERVOIR (TWO SITES)
S23
60 MINUTES
120 MINUTES
REMOVAL OF EXTRACTION RESERVOIR (ONE SITE)
S24
REMOVAL OF EXTRACTION RESERVOIR (ONE SITE)
S25
MEASUREMENT OF GLUCOSE CONCENTRATION AND SODIUM CONCENTRATION
S26
S27
S20
CALCULATION OF PREDICTED BLOOD SUGAR AUC (EQUATION (4))
OBTAINMENT OF INITIAL BLOOD SUGAR VALUE
CALCULATION OF PREDICTED BLOOD SUGAR VALUE (EQUATIONS (5) TO (8.4))
S28
ANALYSIS OF BLOOD SUGAR FLUCTUATION PATTERN
S29

Europäisches Patentamt
European Patent Office
Office européen des brevets

# EUROPEAN SEARCH REPORT

Application Number
EP 10 15 8110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 03/082098 A2 (CYGNUS THERAPEUTIC SYSTEMS [US]) 9 October 2003 (2003-10-09)<br>* page 32, line 5 - page 44, line 20; figures *<br>* page 23, line 4 - page 25, line 29 *<br>* page 29, lines 17-26 *<br>----- | 1-18 | INV.<br>A61B5/00 |
| A | EP 1 839 570 A2 (SYSMEX CORP [JP])<br>3 October 2007 (2007-10-03)<br>* the whole document *<br>----- | 1,13 | |
| | | | TECHNICAL FIELDS SEARCHED (IPC) |
| | | | A61B |

The present search report has been drawn up for all claims

1

| Place of search | Date of completion of the search | Examiner |
|---|---|---|
| Munich | 9 July 2010 | Rosenblatt, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.........................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 10 15 8110

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-2010

| Patent document cited in search report | Publication date | Patent family member(s) | Publication date |
|---|---|---|---|
| WO 03082098 A2 | 09-10-2003 | CA 2480550 A1 | 09-10-2003 |
| | | EP 1489961 A2 | 29-12-2004 |
| | | JP 4083689 B2 | 30-04-2008 |
| | | JP 2005520662 T | 14-07-2005 |
| | | JP 2007252954 A | 04-10-2007 |
| | | JP 2007256296 A | 04-10-2007 |
| | | JP 2007252955 A | 04-10-2007 |
| | | JP 2007252956 A | 04-10-2007 |
| EP 1839570 A2 | 03-10-2007 | JP 2007260315 A | 11-10-2007 |
| | | US 2007232875 A1 | 04-10-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9600110 A